# EUROPEAN PATENT SPECIFICATION

(11) **EP 3 829 579 B1**
(45) Date of publication and mention of the grant of the patent: **02.08.2023**
(21) Application number: 19761959.6
(22) Date of filing: 12.07.2019
(51) Int. Cl.: A61K 31/438, C07D 471/10, A61P 9/10

(54) **1,3,8-TRIAZASPIRO COMPOUNDS AND THEIR USE AS MEDICAMENTS FOR THE TREATMENT OF REPERFUSION INJURY**
1,3,8-TRIAZASPIRO-VERBINDUNGEN UND IHRE VERWENDUNG ALS ARZNEIMITTEL ZUR BEHANDLUNG VON REPERFUSIONSSCHÄDEN
COMPOSÉS DE 1,3,8-TRIAZASPIRO ET LEUR UTILISATION COMME MÉDICAMENTS DESTINÉS AU TRAITEMENT D'UNE LÉSION DE REPERFUSION

(30) Priority: 27.07.2018 IT 201800007580
(43) Date of publication of application: 09.06.2021
(73) Proprietor: Maria Cecilia Hospital S.p.A., 48033 Cotignola (IT)
(72) Inventor: PINTON, Paolo, 44123 Ferrara (IT); FERRARI, Roberto, 44122 Ferrara (IT); GIORGI, Carlotta, 44122 Ferrara (IT); TRAPELLA, Claudio, 44124 Ferrara (IT); PRETI, Delia, 44124 Ferrara (IT); MORCIANO, Giampaolo, 44123 Ferrara (IT)
(74) Representative: Cattaneo, Elisabetta
(86) International application number: PCT/IB2019/055962
(87) International publication number: WO 2020/021378

(56) References cited:
- EP-A1- 1 254 895
- WO-A1-02/083673
- GB-A- 1 043 141
- US-A- 3 155 669
- US-A1- 2005 131 004
- GIAMPAOLO MORCIANO ET AL: "Other bricks for the correct construction of the mitochondrial permeability transition pore complex", CELL DEATH & DISEASE, vol. 8, no. 3, 1 March 2017 (2017-03-01), pages e2698-e2698, XP055571550, DOI: 10.1038/cddis.2017.96
- GIAMPAOLO MORCIANO ET AL: "Discovery of Novel 1,3,8-Triazaspiro[4.5]decane Derivatives That Target the c Subunit of F 1 /F O -Adenosine Triphosphate (ATP) Synthase for the Treatment of Reperfusion Damage in Myocardial Infarction", JOURNAL OF MEDICINAL CHEMISTRY, vol. 61, no. 16, 30 July 2018 (2018-07-30) , pages 7131-7143, XP055571540, US ISSN: 0022-2623, DOI: 10.1021/acs.jmedchem.8b00278
- DANIELI B. ET AL.: "ELECTRON IMPACT MASS SPECTROMETRY OF SUBSTITUTED 1 3 8 TRIAZASPIRO-4 5-DECAN-4-ONES", BIOMEDICAL AND ENVIRONMENTAL MASS SPECTROMETRY, 1989, pages 1000-1004, XP009533710,

## Description

### FIELD OF THE INVENTION

The present invention refers to 1,3,8-triazaspiro compounds, pharmaceutically acceptable salts thereof, and use thereof as medicaments. The compounds of the invention show a marked action against diseases deriving from reperfusion injury. Specifically, the invention provides selective inhibitors of the C subunit of the F₁/F₀-ATP synthase complex. As selective inhibitors, the compounds of the invention are able to modulate the opening of the mitochondrial permeability transition pore in mammalian cells and tissues, and therefore to be used for the treatment of all diseases that show reperfusion damage, such as cardiac, neurological and nephrological diseases.

### STATE OF THE ART

Programmed cell death is an evolutionarily conserved physiological event, essential for intrauterine and post-embryonic development, and for tissue homeostasis through the elimination of damaged cells that otherwise would lead to numerous pathological events (P. Meier, et al. Nature 2000, 407, 796). Conversely, in humans, excessive apoptosis can lead to a long list of pathological dysfunctions, such as cardiovascular, neurological and nephrological disorders. There are several types of programmed cell death, including apoptosis mediated by the transition of mitochondrial permeability (MPT, V. Izzo et al. Trends Cell Biol 2016, 26, 655.) which results in an increase in the permeability of the inner mitochondrial membrane (IMM), usually highly impermeable, with consequent osmotic influx of solutes in the mitochondrial matrix and loss of the structural and functional characteristics of the mitochondria involved (M. Bonora et al. Oncogene 2015, 34, 1608). The MPT state is mediated by the opening of a channel called mitochondrial permeability transition pore (mPTP), a multi-protein platform consisting of proteins, that physically form a pore inside the IMM, and negative and positive modulators that contribute to its functional state and its formation mechanism (A. Halestrap et al. Biochem Soc Trans 2010, 38, 841). Conditions that favour the opening of mPTP include increase in the concentration of calcium ions ([Ca²⁺]) in the mitochondrial matrix, restoration of pH levels, oxidative stress, and the presence of phosphates, all events that occur in relation to injuries due to a ischemia-reperfusion condition (I/R) (G. Morciano et al. J Mol Cell Cardiol 2015, 78, 142).

Although many of the components and modulators of mPTP have been discovered in recent years, the proteins that delimit the channel are still the subject of intense study. Among the mPTP modulators there is the well-known cyclophilin D (CypD), a soluble protein of the mitochondrial matrix (V. Giorgio et al. J Biol Chem 2009, 284, 33982) whose stable depletion has been shown to lead to a protective and desensitization behaviour of MPT. In two recent studies (M. Bonora et al. Cell Cycle 2013, 12, 674; M. Bonora et al. EMBO Rep. 2017,18, 1077), experimental evidence is provided that the subunit C of the F₁/F_{O}-ATP synthase plays a fundamental role in the activity and formation of mPTP, demonstrating in particular a strong correlation between the expression of the C subunit and the functional state of mPTP; in fact, the depletion of this protein leads to a reduction of the channel opening in response to oxidative stress or calcium-induced stress, while its overexpression stimulates the opening (M. Bonora et al. Cell Cycle 2013, 12, 674). Furthermore, we demonstrated that the opening of the mPTP is a multi-step process involving i) the dissociation of F₁/F_{O}-ATP synthase dimers and ii) an appropriate conformation of the protein ring consisting of multiple C subunits once these dimers dissociated. Other groups, such as Alavian's, Azarashvili's and collaborators ones have independently confirmed our first observations by adding important base notions on the properties of the channel (K.N. Alavian et al. Proc Natl Acad Sci U S A 2014, 111, 10580) and the relative phosphorylation state (T. Azarashvili et al. Cell Calcium 2014, 55, 69). The molecular organization of the C subunit in an annular structure (ring C) in IMM causes this to be identified as a key component in the pore assembly process (P.A. et al. Cell Death Discov 2016, 2, 16070; G. Morciano et al. Cell Death Dis 2017, 8, e2698) as well as a promising therapeutic target for the pharmacological treatment of programmed cell death (G. Morciano et al. J Mol Cell Cardiol 2015, 78, 142; R. Ferrari et al. Circ J 2017, 81, 131).

Although numerous efforts have been made to synthesise and test new inhibitors of the mPTP opening (D. Fancelli et al. J Med Chem 2014, 57, 5333; L.J. Martin et al. Front Cell Neurosci 2014, 8, 433), to the best of our knowledge, none of the molecules identified to date has shown specificity of interaction with the C subunit or has been developed for cardioprotective purposes for the treatment of myocardial infarction (MI). It is estimated that approximately 50% of the final infarcted myocardial area is due to reperfusion injury (RI), which consists of cell death following restoration of blood flow in the artery affected by ischemia (R. Ferrari et al. Circ J 2017, 81, 131). To date, only the use of broad-spectrum molecular therapies has demonstrated a useful efficacy in protecting the heart following reperfusion, while randomized clinical trials focused on more specific drug targets have failed.

Compound 8-acetyl-1-phenyl-1,3,8-triazaspiro[4.5]decan-4-one has been described in Danieli et al :"Electron impact mass spectrometry of substituted 1,3,8-triazaspiro-4,5-decan-.4-ones" Biomedical and Environmental mass spectrometry, 1989,pages 1000-1004 as valuable tool for mapping the dopamine receptors in the brain. US-A-2005/131 004 discloses hydronopol derivatives as ORL1 agonists for use in treating *inter alia* myocardial infarction. EP-A-1 254 895 discloses nitrogenous cyclic compounds as calcium antagonists for use in the treatment of *inter alia* neural diseases, including ischemic stroke, cerebral infarction and neural cell death.

The need is therefore felt to have a new pharmacological and/or therapeutic approach for the treatment of pathologies deriving from damages related to reperfusion.

### SUMMARY OF THE INVENTION

The inventors have surprisingly found that some low molecular weight molecules are able to inhibit the opening of mPTP by interacting specifically with the C subunit of the F₁/F_{O}-ATP synthase complex. The invention has therefore allowed the identification of potent inhibitors of the opening of the mitochondrial permeability transition pore in living mammalian cells, that selectively target the C subunit of the F₁/F_{O}-ATP synthase complex. The invention therefore concerns 1,3,8-triazaspiro compounds as defined in the claims which resulted to be able to treat diseases deriving from reperfusion damage.

The invention therefore also concerns a new therapeutic approach comprising the use of cardio-, neuro- and nephroprotective agents as defined in the claims useful for the clinical management of ischemic events related to heart attack and stroke.

The invention therefore concerns a 1,3,8-triazaspiro compound of Formula (I): 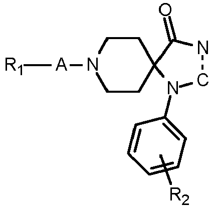 wherein
A is -CH₂, -SO₂-, -NH-CO-, -NH-CS- or -CO-;
the dashed line represents a single or double bond;
R₁ is a substituent selected from the group consisting of (C₁-C₃) alkyl, phenyl, thienyl and cyclohexyl, being said substituent optionally substituted by halogen or (C₁-C₃) alkyl; and
R₂ is a substituent selected from the group consisting of H, (C₁-C₃) alkyl, (C₁-C₃) alkoxy, -CF₃ and halogen; and
wherein
when the dashed line is a double bond, A is -CH₂- and R₁ is phenyl,
or a pharmaceutically acceptable salt thereof
for use as a selective inhibitor of the C subunit of the F₁/F_{O}-ATP synthase complex in the treatment of reperfusion injury diseases.

The invention has therefore provided a compound of Formula (I) as a modulator of the opening of the mitochondrial permeability transition pore in mammalian cells and tissues.

In the present invention when the following definitions are used:
- "(C₁-C₃) alkyl" means linear or branched hydrocarbon chains comprising 1 to 3 carbon atoms;
- "(C₁₋C₃) alkoxy" means a substituent having an (alkyl chain)-O- structure with 1 to 3 carbon atoms; and
- "halogen" means fluorine, chlorine, bromine and iodine.

In another aspect of the invention, the invention concerns a 1,3,8-triazaspiro compound of Formula (I): 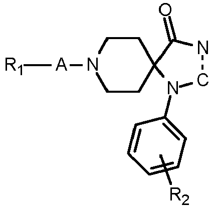 wherein
A is -CH₂, -SO₂-, -NH-CO-, -NH-CS- or -CO-;
the dashed line represents a single or double bond;
R₁ is a substituent selected from the group consisting of (C₁-C₃) alkyl, phenyl, thienyl and cyclohexyl, being said substituent optionally substituted by halogen or (C₁-C₃) alkyl; and
R₂ is a substituent selected from the group consisting of H, (C₁-C₃) alkyl, (C₁-C₃) alkoxy, -CF₃ and halogen; and
wherein
when A is -CH₂, R₁ is phenyl and R₂ is isopropyl, or
when A is -CH₂, R₁ is phenyl, and R₂ is methoxy, then the dashed line is a double bond
for use as a medicament.

In a yet further aspect, the invention concerns a 1,3,8-triazaspiro compound of Formula (I): 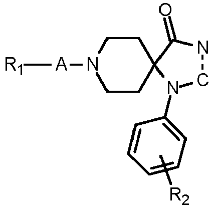 wherein
A is -CH₂, -SO₂-, -NH-CO-, or -NH-CS-;
the dashed line represents a single or double bond;
R₁ is a substituent selected from the group consisting of (C₁-C₃) alkyl, phenyl, thienyl and cyclohexyl, being said substituent optionally substituted by halogen or (C₁-C₃) alkyl;
   and
R₂ is a H, (C₁-C₃) alkyl, (C₁-C₃) alkoxy and halogen substituent; and
wherein
when A is -CH₂- and the dashed line is a single bond, R₁ is phenyl, and R₂ is isopropyl,
when A is -CH₂- and the dashed line is a double bond, R₁ is phenyl, and R₂ is isopropyl or methoxy, and
when A is -SO₂-, R₁ is methyl and R₂ is hydrogen, or R₁ is phenyl or thienyl substituted with halogen, and R₂ is hydrogen or halogen.

In another aspect, the invention concerns a composition comprising a compound of Formula (I) as defined in claim 10 or a pharmaceutically acceptable salt thereof for use as a medicament, and pharmaceutically acceptable additives.

### DESCRIPTION OF THE FIGURES

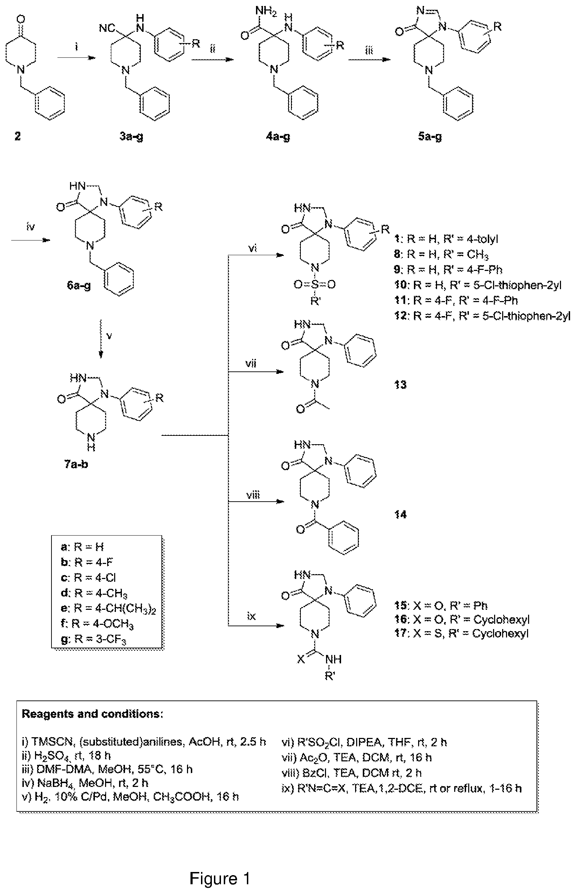

Figure 1 shows the preparation scheme of compounds of Formula (I) for use as medicaments in the treatment of reperfusion damage diseases.

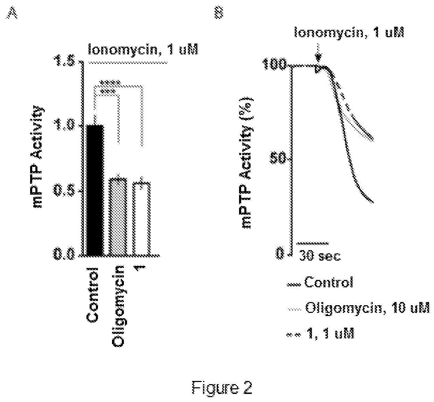

Figure 2 A,B show the results of Example 7 in which the cobalt-calcein assay was performed in untreated (black bar) or pre-treated with Oligomycin A (grey bar) and compound 1 (white bar) HeLa cells. The opening of mPTP was stimulated by administration of lonomycin and the kinetics representative of the different experimental conditions were reported on the side (***: p value<0.001; ****: p value<0.0001).

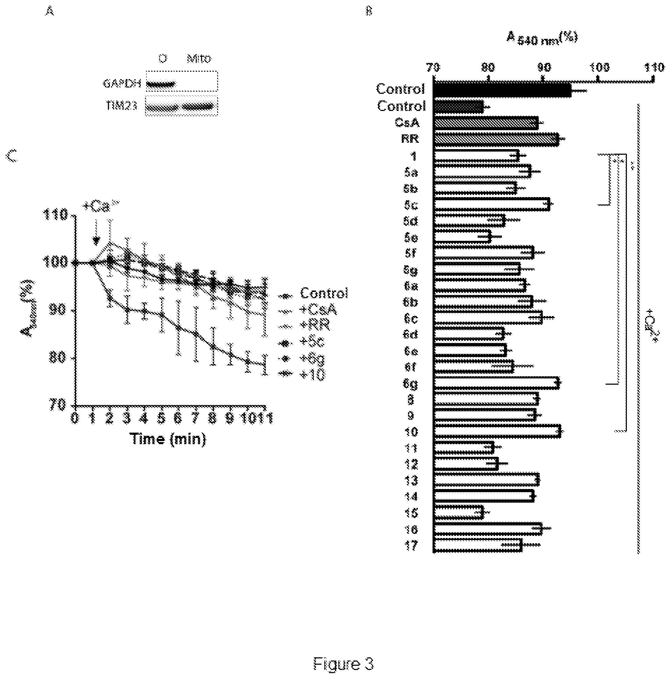

Figure 3, parts A and B, show the results of Example 8, specifically Figure 3A shows the detection of the levels of TIM23 (mitochondrial marker) and GAPDH (cytosolic marker) proteins in the homogenate and mitochondrial portion extracted from liver of mouse; and Figure 3B shows the mitochondrial swelling assay for the screening of synthesized compounds. The data were obtained by recording the absorbance changes at 540 nm at the tenth minute of measurement, and subsequently converted to a percentage. Black bar: untreated and unstimulated mitochondria; black control bar: untreated and Ca2+ stimulated mitochondria; dark grey bar: pre-treatment with CsA and stimulation with Ca2+; light bar: pre-treatment with RR and stimulation with Ca2+; first white bar from the top: treatment with compound 1 and stimulation with Ca2+; white bars: pre-treatment with the compounds of the invention and stimulation with Ca2+. C: kinetics with statistics of the mitochondrial swelling assay under the following conditions: vehicle, CsA, RR, 5c, 6g and 10.

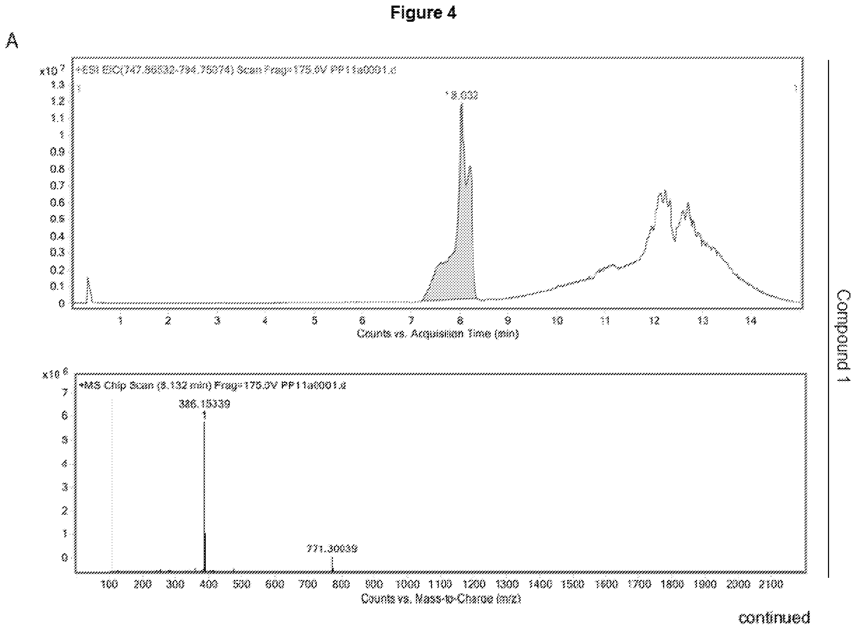

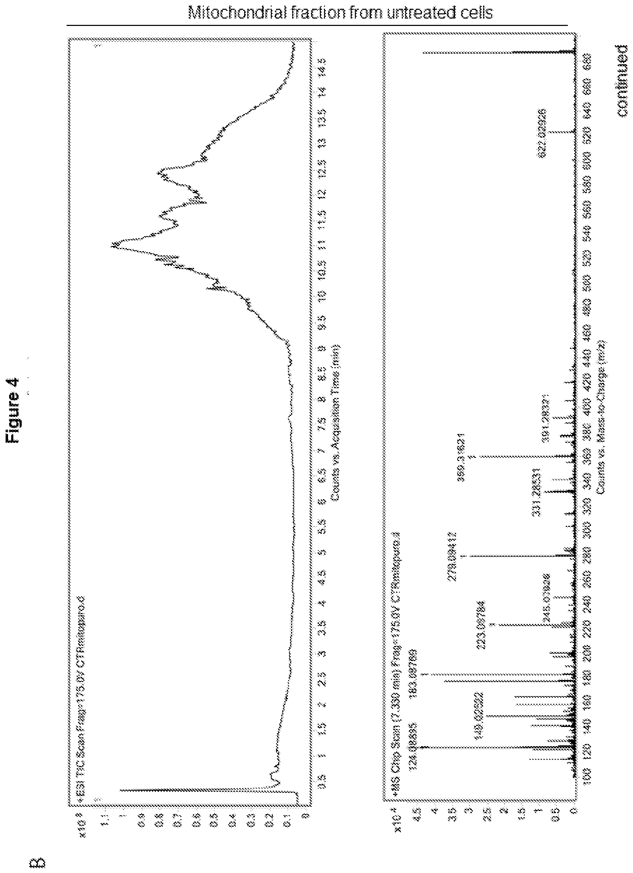

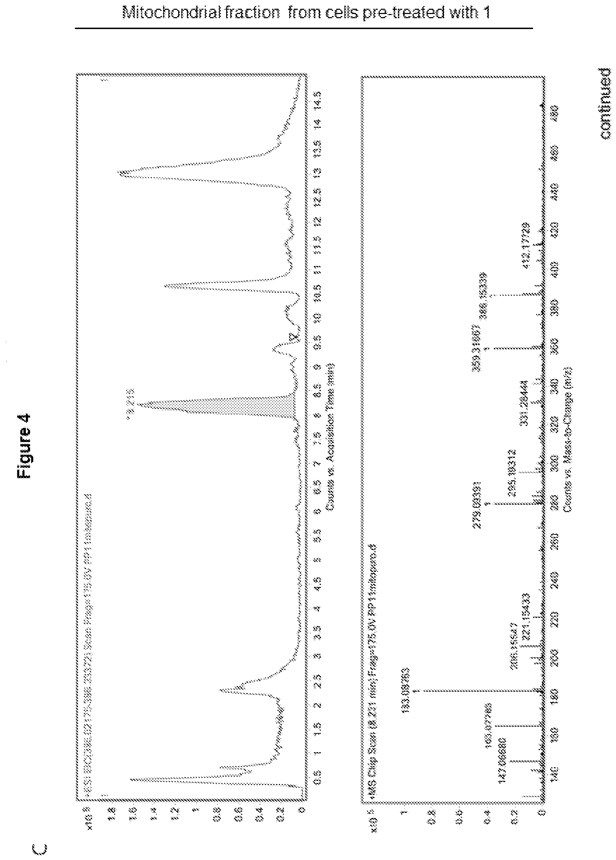

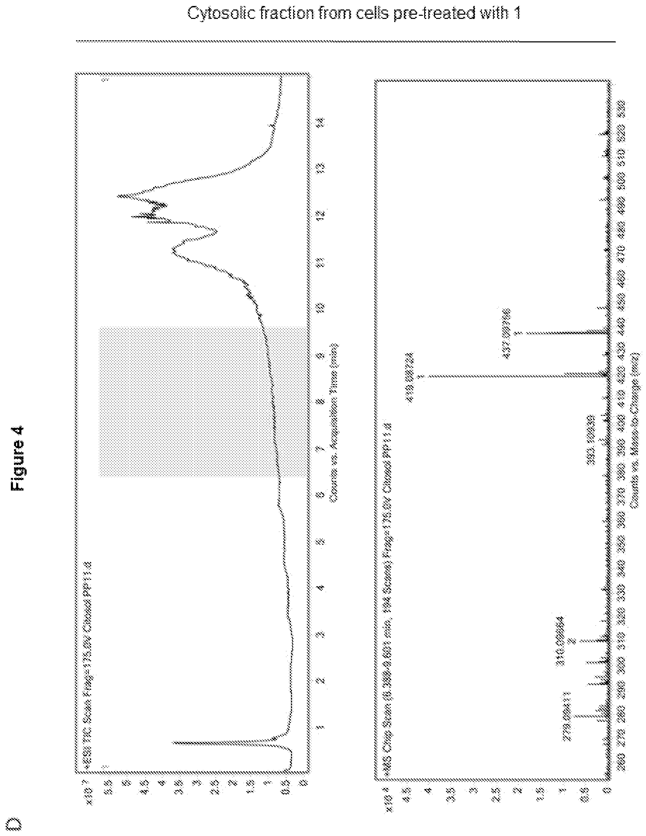

Figure 4 shows the results of Example 9. The subcellular localization of compound 1 was studied by HPLC-HRMS analysis of samples from different cellular compartments, before and after treatment with the compound. A: HPLC-HRMS profile of compound 1 in physiological solution, which revealed a wide absorption band (7-8.5 min) with a maximum at a retention time of 8.03 min (upper panel). The grey region of the HPLC spectrum corresponds to a single mass peak at [M+1]+ m/z of 386.1534 (lower panel, [M+H]+ calculated for compound 1 = 386.1533). B: HPLC profile of the mitochondrial fraction collected from untreated cells which showed a rather clean area in the detection region of compound 1 (from 7 to 9 min, upper panel). The grey band of the HPLC spectrum corresponds to a mixture of mass peaks detected through the HRMS analysis (lower panel). C: HPLC-HRMS profile of the mitochondrial fraction isolated from cells pre-treated with compound 1 which clearly indicates the presence of the compound with an HPLC band at a retention time of 8.2 min (upper panel). The grey area of the signal corresponds to a mixture of mass peaks (lower panel) among which [M+1]+ m/z of 386.1534, which identifies the undetectable compound 1 in the corresponding untreated fraction (panel B). D: No detectable amounts of compound 1 are observed in the cytosolic fraction isolated from cells pre-treated with the compound.

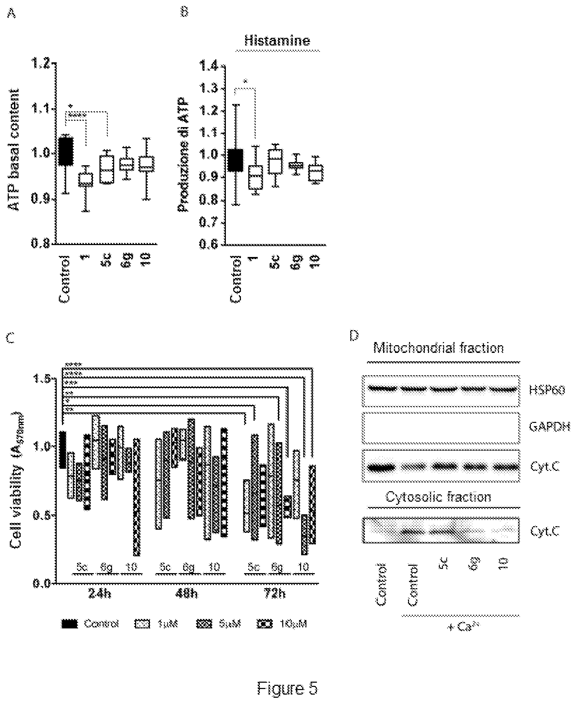

Figure 5 shows the results of Example 10. A: Content of mitochondrial ATP in living cells under basal conditions, evaluated based on the luciferase-luciferin protocol. B: Mitochondrial ATP production following stimulation by a Ca2+ dependent agonist. "Histamine" indicates stimulus with histamine. C: MTT cell viability assay after 24h, 48h and 72h of treatment of live cells with compounds of Formula (I) at different concentrations. In particular, black bar: cells treated with vehicle; dotted bars: compound at the concentration of 1 µM; bars with small black and white squares: compound at a concentration of 5 µM; bars with large black and white squares: compound at a concentration of 10 µM. A standardization was carried out with respect to the vehicle. D: protein analysis for the detection of cytochrome c in the mitochondrial fraction of the different experimental conditions, a.u.: arbitrary units; Cyt.C: cytochrome c; *: p value<0.05; **:p value<0.01; ****:p value<0.0001.

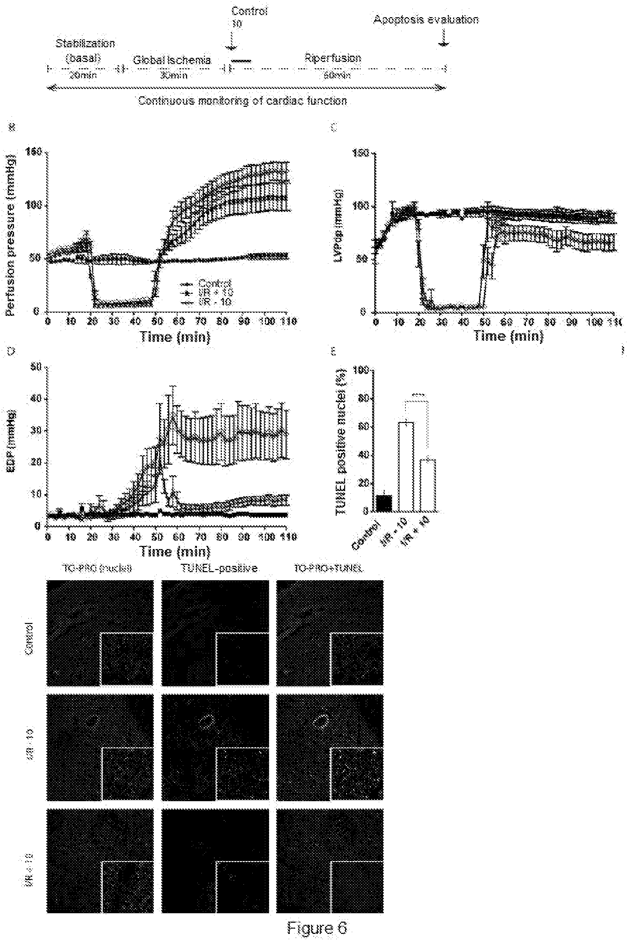

Figure 6 shows the results of Example 11. A: Diagram depicting an experiment on an isolated rat heart by the Langendorff system. B: Coronary artery perfusion pressure (CPP) recorded on rat heart in conditions of stabilization, ischemia and reperfusion. C: Pressure developed by the left ventricle (LVPdp) recorded under the same conditions as B. D: End-diastolic pressure recorded in the same conditions as B and C. E: TUNEL apoptotic assay for the evaluation of the cardioprotective effect in cardiomyocytes of the heart.**:p value<0.01; ****:p value<0.0001.

### DETAILED DESCRIPTION OF THE INVENTION

The invention therefore concerns a 1,3,8-triazaspiro compound of Formula (I): 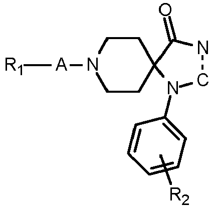 wherein
A is -CH₂, -SO₂-, -NH-CO-, -NH-CS- or -CO-;
the dashed line represents a single or double bond;
R₁ is a substituent selected from the group consisting of (C₁-C₃) alkyl, phenyl, thienyl and cyclohexyl, being said substituent optionally substituted by halogen or (C₁-C₃) alkyl; and
R₂ is a substituent selected from the group consisting of H, (C₁-C₃) alkyl, (C₁-C₃) alkoxy, -CF₃ and halogen; and
wherein
when the dashed line is a double bond, A is -CH₂- and R₁ is phenyl,
or a pharmaceutically acceptable salt thereof
for use as a selective inhibitor of the C subunit of the F₁/F₀-ATP synthase complex in the treatment of reperfusion injury diseases.

The compound is therefore suitable for modulating the opening of the mitochondrial permeability transition pore in mammalian cells and tissues in the treatment of reperfusion injury disorders.

In a first aspect, the invention therefore concerns a 1,3,8-triazaspiro compound or a pharmaceutically acceptable salt thereof as defined in claim 1 for use in the treatment of reperfusion injury diseases.

In this first aspect of the compound of Formula (I), in the first embodiment, A is preferably -SO₂-, the dotted line is a single bond, R₁ is selected from the group consisting of phenyl optionally substituted by halogen or (C₁-C₃) alkyl, thienyl optionally substituted by halogen or (C₁-C₃) alkyl and R2 is hydrogen or halogen. More preferably, when A is -SO₂-, R₁ is selected from phenyl substituted by fluoro or methyl, methyl, and thienyl substituted by chloro, and R₂ is hydrogen or fluoro. Even more preferably, when A is SO₂, R₁ is 4-methylphenyl or 5-chlorothienyl, and R₂ is hydrogen.

In this first aspect of the compound of Formula (I), in the second embodiment, A is preferably -CO-, the dotted line is a single bond, R₁ is selected from the group consisting of phenyl and (C₁-C₃) alkyl, and R₂ is hydrogen. More preferably, when A is -CO-, R₁ is selected from phenyl and methyl.

In this first aspect of the compound of Formula (I), in the third embodiment, A is preferably -NH-CO- or -NHCS-, the dotted line is a single bond, R₁ is selected from the group consisting of phenyl and cyclohexyl, and R₂ is hydrogen.

In this first aspect of the compound of Formula (I), in the fourth embodiment, A is preferably -CH₂-, the dotted line is a single or double bond, R₁ is phenyl, and R₂ is hydrogen, halogen, (C₁-C₃) alkyl, (C₁-C₃) alkoxy and -CF₃. More preferably, when A is -CH₂- and the dotted line is a single or double bond, R₁ is phenyl, and R₂ is hydrogen, chloro, fluoro, methyl, isopropyl, methoxy and -CF₃. Advantageously, in the fourth embodiment, when A is -CH₂- and the dotted line is a double bond, R₁ is phenyl and R₂ is chloro.

In this first aspect, the invention concerns a triazaspiro compound of Formula (I) selected from the group consisting of:
8-benzyl-1-phenyl-1,3,8-triazaspiro[4.5]dec-2-en-4-one (compound 5a) 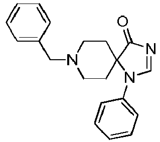
8-benzyl-1-(4-fluorophenyl)-1,3,8-triazaspiro[4.5]dec-2-en-4-one (compound 5b) 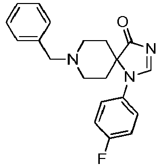
8-benzyl-1-(4-chlorophenyl)-1,3,8-triazaspiro[4.5]dec-2-en-4-one (compound 5c) 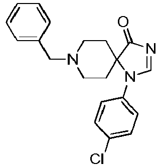
8-benzyl-1-(p-tosyl)-1,3,8-triazaspiro[4.5]dec-2-en-4-one (compound 5d) 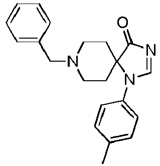
8-benzyl-1-(4-isopropylphenyl)-1,3,8-triazaspiro[4.5]dec-2-en-4-one (compound 5e) 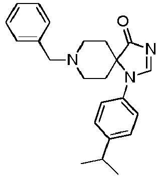
8-benzyl-1-(4-methoxyphenyl)-1,3,8-triazaspiro[4.5]dec-2-en-4-one (compound 5f) 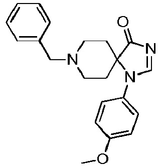
8-benzyl-1-(3-(trifluoromethyl)phenyl)-1,3,8-triazaspiro[4.5]dec-2-en-4-one (compound 5g) 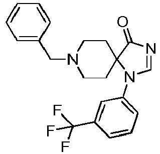
8-benzyl-1-phenyl-1,3,8-triazaspiro[4.5]decan-4-one (compound 6a) 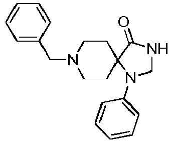
8-benzyl-1-(4-fluorophenyl)-1,3,8-triazaspiro[4.5]decan-4-one (compound 6b) 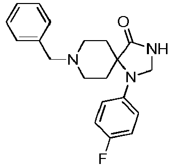
8-benzyl-1-(4-chlorophenyl)-1,3,8-triazaspiro[4.5]decan-4-one (compound 6c) 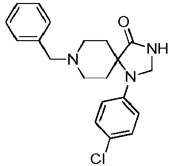
8-benzyl-1-(p-tosyl)-1,3,8-triazaspiro[4.5]decan-4-one (compound 6d) 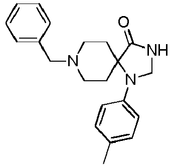
8-benzyl-1-(4-isopropylphenyl)-1,3,8-triazaspiro[4.5]decan-4-one (compound 6e) 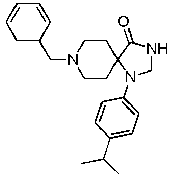
8-benzyl-1-(4-methoxyphenyl)-1,3,8-triazaspiro[4.5]decan-4-one (compound 6f) 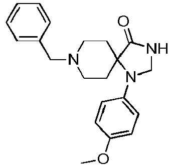
8-benzyl-1-(3-(trifluoromethyl)phenyl)-1,3,8-triazaspiro[4.5]decan-4-one (compound 6g) 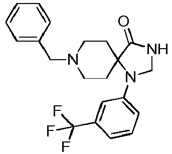
1-phenyl-8-tosyl-1,3,8-triazaspiro[4.5]decan-4-one (compound 1) 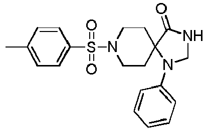
1-phenyl-8-mesyl-1,3,8-triazaspiro[4.5]decan-4-one (compound 8) 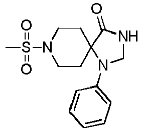
8-((4-fluorophenyl)sulfonyl)-1-phenyl-1,3,8-triazaspiro[4.5]decan-4-one (compound 9) 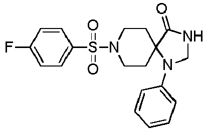
8-((5-chlorotiophen-2-yl)sulfonyl)-1-phenyl-1,3,8-triazaspiro[4.5]decan-4-one (compound 10) 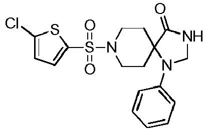
1-(4-fluorophenyl)-8-((4-fluorophenyl)sulfonyl)-1,3,8-triazaspiro[4.5]decan-4-one (compound 11) 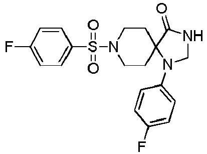
8-((5-chlorotiophen-2-yl)sulfonyl)-1-(4-fluorophenyl)-1,3,8-triazaspiro[4.5]decan-4-one (compound 12) 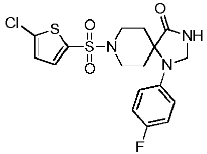
8-acetyl-1-phenyl-1,3,8-triazaspiro[4.5]decan-4-one (compound 13) 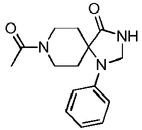
8-benzoyl-1-phenyl-1,3,8-triazaspiro[4.5]decan-4-one (compound 14) 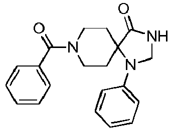
4-oxo-N-1-diphenyl-1,3,8-triazaspiro[4.5]decane-8-carboxamide (compound 15) 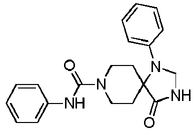
N-cyclohexyl-4-oxo-1-phenyl-1,3,8-triazaspiro[4.5]decane-8-carboxamide (compound 16) 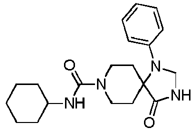
N-cyclohexyl-4-oxo-1-phenyl-1,3,8-triazaspiro[4.5]decane-8-carbothioamide (compound 17)

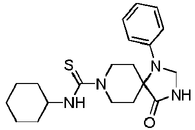

In a second aspect of the invention, the invention concerns a 1,3,8-triazaspiro compound of Formula (I): 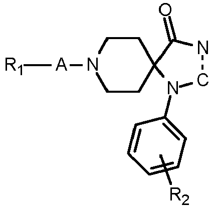 wherein
A is -CH₂, -SO₂-, -NH-CO-, -NH-CS- or -CO-;
the dashed line represents a single or double bond;
R₁ is a substituent selected from the group consisting of (C₁-C₃) alkyl, phenyl, thienyl and cyclohexyl, being said substituent optionally substituted by halogen or (C₁-C₃) alkyl; and
R₂ is a substituent selected from the group consisting of H, (C₁-C₃) alkyl, (C₁-C₃) alkoxy, -CF₃ and halogen; and
wherein
when A is -CH₂, R₁ is phenyl and R₂ is isopropyl,-or
when A is -CH₂, R₁ is phenyl, and R₂ is methoxy, then the dashed line is a double bond
for use as a medicament.

In this second aspect of the compound of Formula (I), in the first embodiment, A is preferably -SO₂-, the dotted line is a single bond, R₁ is selected from the group consisting of phenyl optionally substituted by (C₁-C₃) alkyl, thienyl optionally substituted by (C₁-C₃) alkyl, and R2 is hydrogen or halogen. More preferably, when A is -SO₂, R₁ is selected from phenyl substituted by fluoro or methyl, methyl, and thienyl substituted by chloro, and R₂ is hydrogen or fluoro. Even more preferably, when A is SO₂, R₁ is 4-methylphenyl or 5-chlorothienyl, and R₂ is hydrogen.

In this second aspect of the compound of Formula (I), in the second embodiment, A is preferably -CO-, the dotted line is a single bond, R₁ is selected from the group consisting of phenyl and (C₁-C₃) alkyl, and R₂ is hydrogen. More preferably, when A is -CO-, R₁ is selected from phenyl and methyl.

In this second aspect of the compound of Formula (I), in the third embodiment, A is preferably -NH-CO- or -NHCS-, the dotted line is a single bond, R₁ is selected from the group consisting of phenyl and cyclohexyl, and R₂ is hydrogen.

In this second aspect of the compound of Formula (I), in the fourth embodiment, A is preferably -CH2-, the dotted line is a single or double bond, R₁ is phenyl and R2 is isopropy, or when A is -CH₂, R₁ is phenyl, and R₂ is methoxy, then the dashed line is a double bond.

In the second aspect thereof, the invention concerns a 1,3,8-triazaspiro compound of Formula (I) selected from the group consisting of:
8-benzyl-1-(4-isopropylphenyl)-1,3,8-triazaspiro[4.5]dec-2-en-4-one (compound 5e) 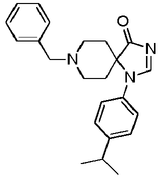
8-benzyl-1-(4-methoxyphenyl)-1,3,8-triazaspiro[4.5]dec-2-en-4-one (compound 5f) 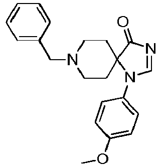
8-benzyl-1-(4-isopropylphenyl)-1,3,8-triazaspiro[4.5]decan-4-one (compound 6e) 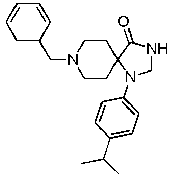
8-benzyl-1-(4-methoxyphenyl)-1,3,8-triazaspiro[4.5]decan-4-one (compound 6f) 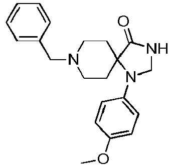
1-phenyl-8-tosyl-1,3,8-triazaspiro[4.5]decan-4-one (compound 1) 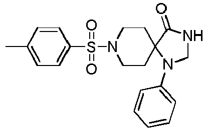
1-phenyl-8-mesyl-1,3,8-triazaspiro[4.5]decan-4-one (compound 8) 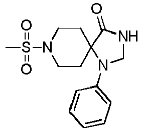
8-((4-fluorophenyl)sulfonyl)-1-phenyl-1,3,8-triazaspiro[4.5]decan-4-one (compound 9) 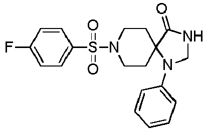
8-((5-chlorotiophen-2-yl)sulfonyl)-1-phenyl-1,3,8-triazaspiro[4.5]decan-4-one (compound 10) 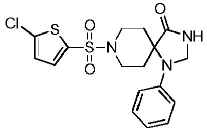
1-(4-fluorophenyl)-8-((4-fluorophenyl)sulfonyl)-1,3,8-triazaspiro[4.5]decan-4-one (compound 11) 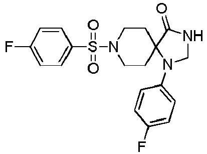
8-((5-chlorotiophen-2-yl)sulfonyl)-1-(4-fluorophenyl)-1,3,8-triazaspiro[4.5]decan-4-one (compound 12) 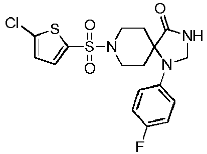
8-acetyl-1-phenyl-1,3,8-triazaspiro[4.5]decan-4-one (compound 13) 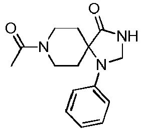
8-benzoyl-1-phenyl-1,3,8-triazaspiro[4.5]decan-4-one (compound 14) 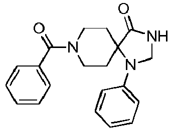
4-oxo-N-1-diphenyl-1,3,8-triazaspiro[4.5]decane-8-carboxamide (compound 15) 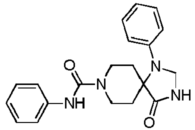
N-cyclohexyl-4-oxo-1-phenyl-1,3,8-triazaspiro[4.5]decane-8-carboxamide (compound 16) 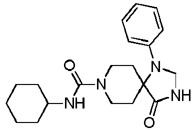
N-cyclohexyl-4-oxo-1-phenyl-1,3,8-triazaspiro[4.5]decane-8-carbothioamide (compound 17)

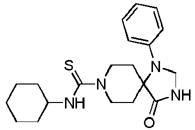

In a third aspect, the invention concerns a 1,3,8-triazaspiro compound of Formula (I): 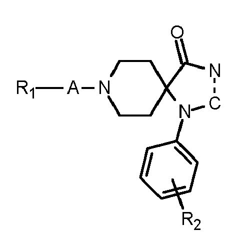 wherein
A is -CH₂-, -SO₂-, -NH-CO-, or -NH-CS-;
the dashed line represents a single or double bond;
R₁ is a substituent selected from the group consisting of (C₁-C₃) alkyl, phenyl, thienyl and cyclohexyl, being said substituent optionally substituted by halogen or (C₁-C₃) alkyl;
   and
R₂ is a substituent selected from the group consisting of H, (C₁-C₃) alkyl, (C₁-C₃) alkoxy and halogen; and
wherein
when A is -CH₂- and the dashed line is a single bond, R₁ is phenyl, and R₂ is isopropyl,
when A is -CH₂- and the dashed line is a double bond, R₁ is phenyl, and R₂ is isopropyl or methoxy, and
when A is -SO₂-, R₁ is methyl and R₂ is hydrogen, or R₁ is phenyl or thienyl substituted with halogen and R₂ is hydrogen or halogen.

In this third aspect of the compound of Formula (I), in the first embodiment, A is preferably -SO₂-, the dotted line is a single bond, R₁ is methyl or phenyl optionally substituted with halogen, and R₂ is hydrogen or halogen. Preferably, when A is - SO₂, R₁ is selected from phenyl substituted by fluoro and thienyl substituted by chloro, and R₂ is fluoro. Even more preferably, when A is -SO₂-, R₁ is 4-fluorophenyl or 5-chlorothienyl, and R₂ is fluoro.

In this third aspect of the compound of Formula (I), in the third embodiment, A is preferably -NH-CO- or -NHCS-, the dotted line is a single bond, R₁ is selected from the group consisting of phenyl and cyclohexyl, and R₂ is hydrogen.

In this third aspect of the compound of Formula (I), in the fourth embodiment, A is preferably -CH₂-, and the dotted line is a single bond, R₁ is phenyl, and R₂ is isopropyl or the dotted line is a double bond, R₁ is phenyl, and R₂ is isopropyl or methoxy.

In the third aspect thereof, the invention concerns a 1 ,3,8-triazaspiro compound of Formula (I) selected from the group consisting of:
8-benzyl-1-(4-isopropylphenyl)-1,3,8-triazaspiro[4.5]dec-2-en-4-one (compound 5e) 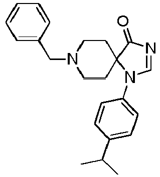
8-benzyl-1-(4-methoxyphenyl)-1,3,8-triazaspiro[4.5]dec-2-en-4-one (compound 5f) 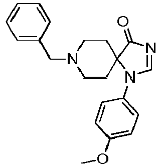
8-benzyl-1-(4-isopropylphenyl)-1,3,8-triazaspiro[4.5]decan-4-one (compound 6e) 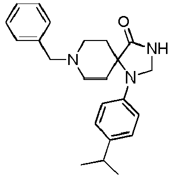
1-phenyl-8-mesyl-1,3,8-triazaspiro[4.5]decan-4-one (compound 8) 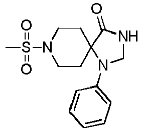
1-(4-fluorophenyl)-8-((4-fluorophenyl)sulfonyl)-1,3,8-triazaspiro[4.5]decan-4-one (compound 11) 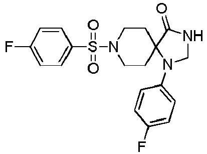
8-((5-chlorotiophen-2-yl)sulfonyl)-1-(4-fluorophenyl)-1,3,8-triazaspiro[4.5]decan-4-one (compound 12) 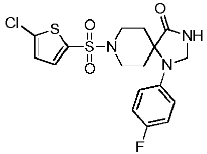
4-oxo-N-1-diphenyl-1,3,8-triazaspiro[4.5]decane-8-carboxamide (compound 15) 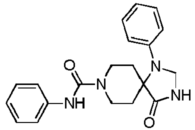
N-cyclohexyl-4-oxo-1-phenyl-1,3,8-triazaspiro[4.5]decane-8-carboxamide (compound 16) 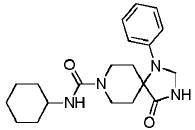
N-cyclohexyl-4-oxo-1-phenyl-1,3,8-triazaspiro[4.5]decane-8-carbothioamide (compound 17)

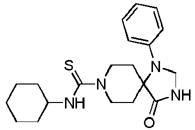

In an even more preferred and advantageous aspect, the invention concerns a compound of Formula (I) selected from the group consisting of:
1-phenyl-8-tosyl-1,3,8-triazaspiro[4.5]decan-4-one (compound 1) 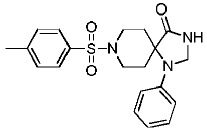
8-benzyl-1-(4-chlorophenyl)-1,3,8-triazaspiro[4.5]dec-2-en-4-one (compound 5c) 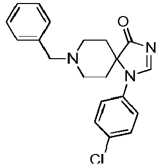
8-benzyl-1-(3-(trifluoromethyl)phenyl)-1,3,8-triazaspiro[4.5]decan-4-one (compound 6g) 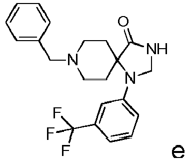
8-((5-chlorotiophen-2-yl)sulfonyl)-1-phenyl-1,3,8-triazaspiro[4.5]decan-4-one (compound 10) 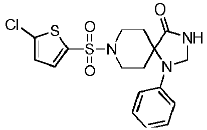
for use in the treatment of reperfusion injury diseases. The invention also more preferably concerns a compound of Formula (I) selected from the group consisting of compound 1 and compound 10 for use as a medicament. Preferably, A is -SO₂- or -CH₂-.

Preferably, R₁ is phenyl or thienyl optionally substituted with halogen or (C₁-C₃) alkyl, more preferably, it is phenyl or thienyl optionally substituted with chloro or methyl.

Preferably, R₂ is hydrogen, -CF₃ or halogen, more preferably the halogen is chloro.

The invention further comprises a pharmaceutically acceptable salt of the compound of Formula (I) selected from the group consisting of hydrochloride, hydrobromide, sulfate, phosphate, acetate, succinate, oxalate, ascorbate, tartrate, gluconate, benzoate, maleate, fumarate and stearate.

The disclosure also includes a compound of Formula (I) labelled with at least one radioisotope such as, for example, tritium (3H), carbon (14C), iodine (1251) or with fluorescent probes, PET (Positron Emission Tomography) or SPECT (Single Photon Emission Tomography) (not part of the claimed invention).

In a further aspect the invention concerns a compound of Formula (I) as defined in claim 10 for use as a medicament and pharmacologically acceptable excipients.

For therapeutic application purposes, the compounds described herein can therefore be suitably formulated for the purposes of administration to mammals (in particular to human subjects), as such or associated in a suitable pharmaceutical composition with one or more pharmaceutically acceptable excipients and/or vehicles. The compositions of the invention include those intended for oral, nasal, sublingual and particularly parenteral (subcutaneous, intramuscular, intravenous and intradermal) administration in the form of aqueous and non-aqueous sterile injectable preparations (solutions or suspensions).

The therapeutic dosage for modulation of the opening of the mitochondrial permeability transition pore by the molecules object of the invention may be extrapolated in vitro using the same molecules on cell lines expressing the cellular target. In view of the biological activity profile shown by the compounds of Formula (I) of the present invention, the pharmaceutical compositions comprising the same can be used for the treatment of diseases and disorders or conditions associated with the need to induce cardioprotection which include heart attack and ischemia-reperfusion injury. In general, the disclosure relates to a method potentially useful for the treatment of diseases mediated by alteration of mPTP functionality (not part of the claimed invention).

The invention will now be exemplified with reference to examples of preparation of compounds of Formula (I) of the invention and evaluation of the therapeutic/medical effects of the compounds as illustrative and not limitative examples.

### EXPERIMENTAL PART

The compounds of the invention were prepared according to the scheme shown in Figure 1.

α-Aminonitrile compounds 3a-g were synthesized by Strecker reaction of N-benzylpiperidone 2 (commercially available) with variously substituted anilines in the presence of trimethyl silyl cyanide (TMSCN). The subsequent treatment with concentrated sulfuric acid led to the amide derivatives (compounds 4a-g) which were then cyclized with dimethylformamide dimethylacetal (DMF-DMA) to give the bicycles (compounds 5a-g). The imidazolinone core was then reduced by treatment with NaBH₄ to obtain the compounds 6a-g. Compounds 6a and 6b were subjected to debenzylation, mediated by hydrogen and catalyzed by Pd/C, to provide the corresponding bicycles, compound 7a and compound 7b. These were used as key intermediates for functionalization of position N8. Specifically, the sulfonamide derivatives, compounds 1 and 8-12, were obtained by treating the compounds 7a-b with suitable sulfonyl chlorides. Acetylation (compound 13) or benzoylation (compound 14) at position N8 were performed by standard treatments in acetic anhydride and benzoyl chloride, respectively. The urea, compounds 15-16, and thiourea (compound 17) derivatives were prepared starting from compound 7a by reaction with suitable isocyanates or isothiocyanates.

### Example 1: Preparation of Compounds 5a-q

### General Procedure for the Synthesis of Compounds 3a-g

To a solution of N1-benzyl-piperidone (1.5 g, 7.9 mmol) in glacial CH₃COOH (20 mL) at 0°C, the appropriate aniline (1 eq) and TMSCN (3 eq) were added. The reaction was continued under stirring at room temperature for 2.5 h, then the mixture was basified (pH > 10) with 2N NaOH. It was suspended with CH₂Cl₂ (20 mL) and the organic phase was washed with water (2 x 10 mL) and brine (1x10 mL). After drying over Na₂SO₄, the organic solvent was evaporated until a solid residue was obtained, which was triturated with Et₂O and filtered to give the compounds 3a-g as pale yellow solids, that were used in the subsequent reactions without further purifications.

### General Procedure for the Synthesis of 4a-g

Compounds 3a-g (4.8 mmol) were dissolved in H₂SO₄ (96% w/w, 7 mL) and the resulting mixture was stirred at room temperatures for 18 h. The reaction was then basified (pH > 10) by gradual addition of NH₄OH (30% w/w), while keeping the temperature below 0 °C. The aqueous phase was extracted with CH₂Cl₂ (20 mL) and the organic extract washed with water (2 x 10 mL) and brine (1 x 10 mL). After drying over Na₂SO₄, the organic solvent was evaporated until a solid residue was obtained, which was triturated with Et₂O and filtered to give the compounds 4a-g as pale yellow solids that were used in the subsequent reactions without further purification.

### General Procedure for the Synthesis of 5a-g

To a solution of compounds 4a-g (4.8 mmol) in MeOH (10 mL) DMF-DMA (3 eq) was added, and the mixture obtained was heated at 55°C for 16 h. The solvents were evaporated until a solid residue was obtained, which was crystallized with Et₂O to give the bicycles, compounds 5a-g, as white solids.

8-benzyl-1-phenyl-1,3,8-triazaspiro[4.5]dec-2-en-4-one (5a).

Yield: 92%; mp: 158-159 °C; ¹H NMR (400 MHz, CDCl₃) δ 8.24 (s, 1H), 7.48-7.43 (m, 3H), 7.35-7.11 (m, 7H), 3.55 (s, 2H), 3.13-2.95 (m, 2H), 2.79-2.65 (m, 2H), 2.15-1.95 (m, 2H), 1.81-1.78 (m, 2H).

8-benzyl-1-(4-fluorophenyl)-1,3,8-triazaspiro[4.5]dec-2-en-4-one (5b).

Yield: 90%; mp 224-225 °C; ¹H NMR (400 MHz, CDCl₃) δ 8.19 (s, 1H), 7.40-7.21 (m, 4H), 7.17-6.98 (m, 5H), 3.59 (s, 2H), 3.02-2.98 (m, 2H), 2.72-2.68 (m, 2H), 1.89-1.84 (m, 2H), 1.80-1.77 (m, 2H).

8-benzyl-1-(4-chlorophenyl)-1,3,8-triazaspiro[4.5]dec-2-en-4-one (5c).

Yield: 88%; mp 224-226 °C; ¹H NMR (400 MHz, CDCl₃) δ 8.23 (s, 1H), 7.47-7.32 (m, 2H), 7.31-7.25 (m, 5H), 7.15-7.12 (m, 2H), 3.58 (s, 2H), 3.14-2.94 (m, 2H), 2.78-2.71 (m, 2H), 2.10-2.01 (m, 2H), 1.78 (d, J = 13.5 Hz, 2H).

8-benzyl-1-(p-tosyl)-1,3,8-triazaspiro[4.5]dec-2-en-4-one (5d).

Yield: 88%; mp 221-223 °C; ¹H NMR (400 MHz, CDCl₃) δ 8.20 (s, 1H), 7.35-7.28 (m, 6H), 7.06 (d, J = 8.1 Hz, 3H), 3.53 (s, 2H), 3-11-2.99 (m, 2H), 2.73-2.68 (m, 2H), 2.40 (s, 3H), 2.11-1.97 (m, 2H), 1.72-1.69 (m, 2H).

8-benzyl-1-(4-isopropylphenyl)-1,3,8-triazaspiro[4.5]dec-2-en-4-one (5e).

Yield: 82%; mp 139-140 °C; ¹H NMR (400 MHz, CDCl₃) δ 8.20 (s, 1H), 7.44-7.22 (m, 7H), 7.11-7.01 (d, J = 8.00 Hz, 2H), 3.59 (s, 2H), 3.01-2.97 (m, 3H), 2.80-2.70 (m, 2H), 2.05-1.98 (m, 2H), 1.78 (d, J = 12.4 Hz, 2H), 1.27 (d, J = 6.9 Hz, 6H). 8-benzyl-1-(4-methoxyphenyl)-1,3,8-triazaspiro[4.5]dec-2-en-4-one (5f).

Yield: 92%; mp 200-201 °C; ¹H NMR (400 MHz, CDCl₃) δ 8.17 (s, 1H), 7.18-7.22 (m, 5H), 7.15 (d, J = 8.0 Hz, 2H), 6.89 (d, J = 8 Hz, 2H), 3.84 (s, 3H), 3.59 (s, 2H), 3.15-3.01 (m, 2H), 2.78-2.68 (m, 2H), 2.01-1.98 (m, 2H), 1.83-1.78 (m, 2H).

8-benzyl-1-(3-(trifluoromethyl)phenyl)-1,3,8-triazaspiro[4.5]dec-2-en-4-one (5 g). Yield: 26%; mp 154-155 °C; ¹H NMR (400 MHz, CDCl₃) δ 8.29 (s, 1H), 7.75 (d, J = 8 Hz, 1H), 7.65 (t, J = 8 Hz, 1H), 7.43-7.27 (m, 7H), 3.62 (s, 2H), 3.11-3.01 (m, 2H), 2.84-2.74 (m, 2H), 2.19-1.66 (m, 4H).

### Example 2: Preparation of Compounds 6a-g.

To a solution of compounds 5a-g (3.4 mmol) in MeOH (15 mL), NaBH₄ (1.25 eq) was added, and the resulting mixture was stirred at room temperature for 2 h. Water was carefully added, then the volume of the reaction was halved on a rotavapor and the resulting residue was extracted with EtOAc (3 x 15 mL). The organic extract was then washed with water (1 x 10 mL) and brine (1 x 10 mL) and dried over Na₂SO₄. The solvent was finally evaporated until a solid residue was obtained, which was crystallized with MeOH to give the compounds 6a-g as white solids.

8-benzyl-1-phenyl-1,3,8-triazaspiro[4.5]decan-4-one (6a).

Yield: 96%; mp 158-160 °C; ¹H NMR (400 MHz, DMSO-d₆) δ 8.65 (s, 1H), 7.42-7.32 (m, 4H), 7.28-7.23 (m, 3H), 6.86 (d, J = 8.2 Hz, 2H), 6.76 (t, J = 7.2 Hz, 1H), 4.57 (s, 2H), 3.52 (s, 2H), 2.83-2.36 (m, 6H), 1.57 (d, J = 13.5 Hz, 2H). 8-benzyl-1-(4-fluorophenyl)-1,3,8-triazaspiro[4.5]decan-4-one (6b).

Yield: 84%; mp 214-216 °C; ¹H NMR (400 MHz, CDCl₃) δ 7.34 - 7.25 (m, 6H), 7.04 - 6.93 (m, 4H), 4.68 (s, 2H), 3.58 (bs, 2H), 2.77 (bs, 4H), 2.34 (bs, 2H), 1.75 (d, J = 14.2 Hz, 2H).

8-benzyl-1-(4-chlorophenyl)-1,3,8-triazaspiro[4.5]decan-4-one (6c).

Yield 79%; mp 231-232 °C; ¹H NMR (400 MHz, DMSO-d₆) δ 8.68 (s, 1H), 7.36-7.24 (m, 7H), 6.85 (d, J = 9.1 Hz, 2H), 4.56 (s, 2H), 3.52 (s, 2H), 2.71 (d, J = 7.3 Hz, 4H), 2.53-2.47 (m, 2H), 1.57 (d, J = 13.6 Hz, 2H).

8-benzyl-1-(p-tosyl)-1,3,8-triazaspiro[4.5]decan-4-one (6d).

Yield: 85%; mp 226-227 °C; ¹H NMR (400 MHz, DMSO-d₆) δ 8.58 (s, 1H), 7.33-7.32 (m, 4H), 7.27- 7.21 (m, 1H), 7.07 (d, J = 8.3 Hz, 2H), 6.81 (d, J = 8.6 Hz, 2H), 4.52 (s, 2H), 3.49 (s, 2H), 2.70 (m, 4H), 2.40-2.30 (m, 2H), 2.21 (s, 3H), 1.56 (d, J = 13.7 Hz, 2H).

8-benzyl-1-(4-isopropylphenyl)-1,3,8-triazaspiro[4.5]decan-4-one (6e).

Yield: 45%; mp 189-190 °C; ¹H NMR (400 MHz, CDCl₃) δ 7.36- 7.17 (m, 7H), 6.92-6.83 (m, 3H), 4.72 (s, 2H), 3.64-3.51 (m, 2H), 2.89-2.73 (m, 4H), 2.57 (bs, 1H), 2.17 (s, 2H), 1.74-171 (m, 2H), 1.23 (d, J = 6.9 Hz, 6H).

8-benzyl-1-(4-methoxyphenyl)-1,3,8-triazaspiro[4.5]decan-4-one (6f).

Yield: 83%; mp 205-207 °C; ¹H NMR (400 MHz, DMSO-d₆) δ 8.49 (s, 1H), 7.32-7.20 (m, 5H), 6.96 (d, J = 8.0 Hz, 2H), 6.88 (d, J = 8.0 Hz, 2H), 4.49 (s, 2H), 3.71 (s, 3H), 3.45 (s, 2H), 2.70-2.54 (m, 4H), 2.00-1.95 (m, 2H), 1.62 (d, J = 13.7 Hz, 2H).

8-benzyl-1-(3-(trifluoromethyl)phenyl)-1,3,8-triazaspiro[4.5]decan-4-one (6g). Yield: 76%; mp 198-200 °C; ¹H NMR (400 MHz, CDCl₃) δ 7.40-7.26 (m, 6H), 7.12-7.07 (m, 4H), 4.78 (s, 2H), 3.60 (s, 2H), 2.83-2.73 (m, 6H), 1.72 (d, J = 12.6 Hz, 2H).

### Example 3: Preparation of Compounds 1, 8-12:

### General Procedure for the Synthesis of 7a-b

Compounds 6a and 6b (1 mmol) were dissolved in MeOH (30 mL) and glacial CH₃COOH (1 mL), the solution was treated with a catalytic amount (0.1 mmol) of palladium on carbon (10% Pd) and placed in a hydrogen atmosphere. After 36 h, the mixture was filtered through celite and the filtrate was concentrated under reduced pressure. The residue was taken up with water and basified by adding a saturated aqueous solution of NaHCOs. The solution was extracted with CH₂Cl₂ (4 x 10 mL) and the organic extracts were combined and dried over Na₂SO₄. After filtration, the organic solvent was evaporated to give the compounds 7a and 7b as white solids.

### 1-phenyl-1,3,8-triazaspiro[4.5]decan-4-one (7a)

Yield: 86%; mp 172-173 °C; ¹H NMR (400 MHz, DMSO-d₆) δ 8.78 (s, 1H), 7.29-7.23 (m, 2H), 6.82-6.79 (m, 3H), 4.58 (s, 2H), 3.18-3.16 (m, 2H), 2.95-2.91 m, 2H), 2.34-2.28 (m, 2H), 1.55-1.46 (m, 2H); MS (ESI): [MH]+ = 232.16. 1-(4-fluorophenyl)-1,3,8-triazaspiro[4.5]decan-4-one (7b)

Yield: 78%; ¹H NMR (400 MHz, DMSO-d₆) δ 8.72 (s, 1H), 7.09-7.03 (m, 2H), 6.97-6.93 (m, 2H), 5.4 (bs, 1H), 4.55 (s, 2H), 3.26-3.19 (m, 2H), 2.97-2.93 m, 2H), 2.38-2.30 (m, 2H), 1.60-1.56 (m, 2H). MS (ESI): [MH]+ = 250.39.

### Procedure for the Synthesis of 1, 8-12

To a solution of compounds 7a and 7b (0.4 mmol) in THF (5 mL), DIPEA (1 eq) and the appropriate sulfonyl chloride (1.2 eq) were added. The mixture was stirred at room temperature for 2 h, then diluted with EtOAc and the organic phase was washed with water (1 × 5 mL) and brine (1 × 5 mL). The organic extract was dried over Na₂SO₄ and, after filtration, the solvent was evaporated to give a solid residue which was purified by chromatographic column (100% EtOAc) to give the final derivatives 1, 8-12 as white solids.

### 1-phenyl-8-tosyl-1,3,8-triazaspiro[4.5]decan-4-one (1).

Yield: 72%; mp 250-251 °C; ¹H NMR (400 MHz, DMSO-d₆) δ 8.78 (s, 1H), 7.66 (d, J = 8.2 Hz, 2H), 7.47 (d, J = 8.1 Hz, 2H), 7.23 (t, J = 7.9 Hz, 2H), 6.80-6.71 (m, 3H), 4.56 (s, 2H), 3.65 (d, J = 6.8 Hz, 2H), 3.04-2.99 (m, 2H), 2.52-2.35 (m, 5H), 1.68 (d, J = 13.8 Hz, 2H).

### 1-phenyl-8-mesyl-1,3,8-triazaspiro[4.5]decan-4-one (8).

Yield: 87%; mp 248-249 °C; ¹H NMR (400 MHz, DMSO-d₆) δ 8.90 (s, 1H), 7.25 (t, J = 8.0 Hz, 2H), 6.83-6.78 (m, 3H), 4.61 (s, 2H), 3.59-3.55 (m, 2H), 3.44-3.37 (m, 2H), 2.92 (s, 3H), 2.58-2.51 (m, 2H), 1.74 (d, J = 13.9 Hz, 2H). 8-((4-fluorophenyl)sulfonyl)-1-phenyl-1,3,8-triazaspiro[4.5]decan-4-one (9). Yield: 78%; mp 251-252 °C; ¹H NMR (400 MHz, DMSO-d₆) δ 8.77 (s, 1H), 7.89-7.82 (m, 2H), 7.51 (t, J = 8.8 Hz, 2H), 7.23 (t, J = 8.0 Hz, 2H), 6.81-6.70 (m, 3H), 4.56 (s, 2H), 3.68 (dd, J = 11.4, 4.8 Hz, 2H), 3.10-3.04 (m, 2H), 2.49-2.48 (m, 2H), 1.69 (d, J = 13.8 Hz, 2H).

8-((5-chlorotiophen-2-yl)sulfonyl)-1-phenyl-1,3,8-triazaspiro[4.5]decan-4-one (10).

Yield: 71%; mp 258-259 °C; ¹H NMR (400 MHz, DMSO-d₆) δ 8.82 (s, 1H), 7.61 (d, J = 4.1 Hz, 1H), 7.41 (d, J = 4.1 Hz, 1H), 7.25 (dd, J = 8.6, 7.4 Hz, 2H), 6.81-6.73 (m, 3H), 4.58 (s, 2H), 3.66 (dd, J = 11.4, 4.9 Hz, 2H), 3.21 (dd, J = 12.2, 9.3 Hz, 2H), 2.55-2.49 (m, 2H), 1.74 (d, J = 14.1 Hz, 2H).

1-(4-fluorophenyl)-8-((4-fluorophenyl)sulfonyl)-1,3,8-triazaspiro[4.5]decan-4-one (11).

Yield: 51%; mp 259-260 °C; ¹H NMR (400 MHz, DMSO-d₆) δ 8.74 (s, 1H), 7.87-7.82 (m, 2H), 7.52-7.47 (m, 2H), 7.12-7.08 (m, 2H), 6.83-6.80 (m, 2H), 4.52 (s, 2H), 3.61 (d, J = 7.0 Hz, 2H), 3.05 (td, J = 12.2, 2.9 Hz, 2H), 2.21 (td, J = 13.5, 5.2 Hz, 2H), 1.72 (d, J = 14.0 Hz, 2H).

8-((5-chlorotiophen-2-yl)sulfonyl)-1-(4-fluorophenyl)-1,3,8-triazaspiro[4.5]decan-4-one (12).

Yield: 75%; mp 244-245 °C; ¹H NMR (400 MHz, DMSO-d₆) δ 8.79 (s, 1H), 7.60 (dd, J = 10.4, 4.1 Hz, 1H), 7.41 (dd, J = 8.1, 4.1 Hz, 1H), 7.15-7.08 (m, 2H), 6.87-6.81 (m, 2H), 4.55 (s, 2H), 3.60 (d, J = 6.7 Hz, 2H), 3.20 (td, J = 12.1, 2.9 Hz, 2H), 2.27 (td, J = 13.5, 5.2 Hz, 2H), 1.77 (d, J = 14.1 Hz, 2H).

### Example 4: Preparation of the Compound 8-acetyl-1-phenyl-1,3,8-triazaspiro[4.5]decan-4-one (13)

To a solution of compound 7a (0.4 mmol) in CH₂Cl₂ (25 mL), TEA (1 eq) and Ac₂O (1 eq) were added, and the mixture was stirred at room temperature for 3 h. It was diluted with CH₂Cl₂ (30 mL) and the organic phase was washed with 1M HCl (1x10 mL), water (1x10 mL) and brine (1x10 mL). It was dried over sodium sulfate, filtered and the solvent concentrated. The residue was purified by chromatographic column (EtOAc 100%) to give compound 13 as a white solid. Yield: 93%; mp 197-198 °C; ¹H NMR (400 MHz, CD₃OD) δ 7.26-7.22 (m, 2H), 6.85-6.81 (m, 3H), 4.88 (s, 2H), 4.41-4.36 (m, 1H), 3.89-3.86 (m, 2H), 3.45 (td, J = 12.8, 3.6 Hz, 1H), 2.54-2.39 (m, 2H), 2.14 (s, 3H), 1.83-1.78 (m, 2H).

### Example 5: Preparation of the Compound 8-benzoyl-1-phenyl-1,3,8-triazaspiro[4.5]decan-4-one (14)

To a solution of compound 7a (0.4 mmol) in CH₂Cl₂ (10 mL), TEA (1 eq) and benzoyl chloride (1 eq) were added, and the mixture was stirred at room temperature for 2 h. The mixture was diluted with CH₂Cl₂ (30 mL) and washed with 1M HCl (1x10 mL), water (1×10 mL) and brine (1×10 mL). It was diluted with CH₂Cl₂ (30 mL) and the organic phase was washed with 1M HCl (1x10 mL), water (1x10 mL) and brine (1x10 mL). It was dried over sodium sulfate, filtered and the solvent concentrated. The residue was purified by chromatographic column (EtOAc 100%) to give compound 14 as a white solid.

Yield: 80%; mp 215-217 °C; ¹H NMR (400 MHz, CDsOD) δ 7.49-7.45 (m, 5H), 7.31-7.26 (m, 2H), 6.89-6.86 (m, 3H), 4.70 (d, J = 7.6 Hz, 2H), 4.52 (d, J = 11.2 Hz, 1H), 3.87 (t, J = 11.8 Hz, 1H), 3.67 (t, J = 11.4 Hz, 2H), 2.61 (td, J = 13.4, 5.2 Hz, 1H), 2.39 (td, J = 13.5, 5.0 Hz, 1H), 1.86 (d, J = 13.8 Hz, 1H), 1.68 (d, J = 14.1 Hz, 1H).

### Example 6: Preparation of Compounds 15-17

### Preparation of Compounds 15-16.

To a solution of compound 7a (0.4 mmol) in CH₂Cl₂ (20 mL), the appropriate isocyanate (1 eq) was added, and the mixture was stirred at room temperature for 16 h. The organic solvent was evaporated until a solid residue was obtained, which was partitioned between water (10 mL) and EtOAc (10 mL). The aqueous phase was further extracted with EtOAc (2x10 mL) and the combined extracts were dried over Na₂SO₄. After filtration, the solvent was evaporated, and the residue purified by chromatographic column (EtOAc) to give compounds 15 and 16 as white solids.

4-oxo-N-1-diphenyl-1,3,8-triazaspiro[4.5]decane-8-carboxamide (15).

Yield: 72%; mp 250-251 °C; ¹H NMR (400 MHz, DMSO-d₆) δ 8.79-8.65 (m, 2H), 7.45-7.44 (m, 2H), 7.24-7.15 (m, 4H), 6,94-6.92 (m, 1H), 6.70-6.69 (m, 2H), 4.59 (s, 2H), 4.03 (bs, 2H), 3.48 (bs, 2H), 2.47 (bs, 3H), 1.60 (d, J = 12.5 Hz, 2H). N-cyclohexyl-4-oxo-1-phenyl-1,3,8-triazaspiro[4.5]decane-8-carboxamide (16). Yield: 65%, mp: 253-254 °C; ¹H NMR (400 MHz, DMSO-d₆) δ 8.74 (s, 1H), 7.13 (t, J = 7.9 Hz, 2H), 6.72-6.60 (m, 3H), 6.33 (d, J = 7.6 Hz, 1H), 4.57 (s, 2H), 3.88 (dd, J = 12.8, 3.9 Hz, 2H), 3.49-3.47 (m, 1H), 2.43-2.37 (m, 4H), 1.78-1.67 (m, 4H), 1.58-1.47 (m, 2H), 1.44-1. (m, 6H).

### Preparation of Compound 17

To a solution of compound 7a (0.4 mmol) in 1,2-dichloroethane (20 mL), TEA (1 eq) and isothiocyanate (1 eq) were added, and the mixture was stirred at room temperature for 18 h. It was diluted with CH₂Cl₂ and the organic phase was washed with 2N HCl, water and brine. After drying over Na₂SO₄, the organic solvent was filtered and evaporated to obtain a residue that was purified by chromatographic column (Petroleum ether/EtOAc 1:2) until compound 17 was obtained as a white solid.

N-cyclohexyl-4-oxo-1-phenyl-1,3,8-triazaspiro[4.5]decane-8-carbothioamide (17)

Yield: 72%; mp: 247-248 °C; ¹H NMR (400 MHz, DMSO-d₆) δ 8.81 (s, 1H), 7.36 (d, J = 8.0 Hz, 1H), 7.15-7.11 (m, 2H), 6.70 (t, J = 8.0 Hz, 1H), 6.58 (d, J = 8.0 Hz, 2H), 4.58-4.49 (m, 4H), 4.28 (bs, 1H), 3.62 (dd, J = 12.7, 10.2 Hz, 2H), 2.53-3.50 (m, 2H), 1.87-1.52 (m, 6H), 1.29-0.99 (m, 6H).

### Example 7: Evaluation of inhibition of mPTP opening in vitro

The compound 1-phenyl-8-tosyl-1,3,8-triazaspiro[4.5]decan-4-one (Compound 1) and compounds of formula (I) are able to inhibit the mPTP opening in vitro. In order to determine the effect of 1-phenyl-8-tosyl-1,3,8-triazaspiro[4.5]decan-4-one (compound 1) on the Ca²⁺-induced mPTP opening, a cobalt-calcein assay in HeLa cells (M. Bonora et al. Nat Protoc 2016, 11, 1067) was performed. The mPTP opening was stimulated by addition of the ionophore ionomycin and the resulting kinetics were compared. As shown in Figure 2A, pre-treatment with compound 1 (1 µM) resulted in about 50% desensitization of the mPTP opening, as assessed by the kinetics slope following stimulation, which was significantly lower compared to untreated cells (Figure 2B). Furthermore, it was demonstrated that compound 1 was able to inhibit the mPTP opening in a similar way to Oligomycin (C. Chinopoulos et al. Front Oncol 2013, 3, 25; J. Symersky et al. Proc Natl Acad Sci U S A 2012, 109, 13961; L. Blanchet et al. PLoS One 2014, 9, e114090), but at a concentration ten times lower. These data showed that compound 1 is able to inhibit the opening of mPTP more effectively than the known inhibitor Oligomycin. To study the efficacy of inhibition of the compounds of Formula (I) in terms of mPTP opening, the previously described mitochondrial swelling assay (F. Di Lisa et al. J Biol Chem 2001, 276, 2571) was used, being a faster protocol than the corresponding microscopic analysis for screening multiple compounds. All the compounds prepared above resulted to be able to desensitize the opening of mPTP.

### Example 8: Evaluation of effects of the compounds

To demonstrate the specificity in measuring Ca²⁺-induced mPTP opening, we added two positive controls, such as using cyclosporine A (CsA) and Red Ruthenium (RR): the first is a known inhibitor of mPTP, while the second is a mitochondrial calcium accumulation blocker. Mitochondria isolated from mouse liver were checked for purity (Figure 3A) and functionality thereof, and then stimulated with Ca²⁺; the decrease in absorbance at 540 nm, indicative of mitochondrial swelling, was monitored for 10 minutes. Under these conditions, the mitochondria treated with the vehicle showed a consistent swelling of the organelle, while this process was prevented by pre-treatment with CsA and RR (Figure 3B,C). As shown in Figure 3B, most of the compounds of the invention demonstrated good inhibition potency when compared to the mitochondria control sample (black bar). In particular, compounds 5c, 6g and 10 demonstrated to be significantly more performing than compound 1 as they brought the mitochondria back to a physiological condition (panel B, black bar), based on structural and functional point of view.

### Example 9: Evaluation of effects of the compounds

1-Phenyl-8-tosyl-1,3,8-triazaspiro[4.5]decan-4-one (compound 1) accumulated in the mitochondria and bound the C subunit of the F1/FO-ATP synthase complex. To monitor and better characterize the biological activity of compound 1, its subcellular localization was studied with greater interest for the mitochondrial compartment, hypothetical site of action. Subcellular fractionation was performed (J. M. Suski et al. Nat Protoc 2014, 9, 312) in an attempt to isolate pure mitochondria, cytosol and endoplasmic reticulum fractions starting from cells pre-treated or not with compound 1. Representative samples of each subcellular compartment were subjected to HPLC-HRMS analysis for compound detection. As shown in Figure 4, the pre-treatment with compound 1 led to selective accumulation of the compound in the mitochondria without detectable traces in the other subcellular fractions. In addition, to highlight the intracellular target of compound 1, a Cellular Thermal Shift Assay (CETSA) was carried out (R. Jafari et al. Nat Protoc 2014, 9, 2100; D. Martinez Molina et al. Science 2013, 341, 84), a protocol based on the thermal stabilization induced by the ligand in the target protein, in this case the subunit C of the F1/FO-ATP synthase. Since thermal shifts at high ligand concentrations correlated with the average IC₅₀ values and affinities measured by other methods (O. Fedorov et al. Proc Natl Acad Sci U S A 2007, 104, 20523; E. Wahlberg et al. Nat Biotechnol 2012, 30, 283), it was possible to establish the selective binding of compound 1 to the subunit C of the F1/FO-ATP synthase between 53 and 65 °C. Together these data confirmed the ability of compound 1 to selectively enter the mitochondria and to bind to th subunit C to exercise mPTP inhibition.

### Example 10: Cytotoxicity profile of molecules 1, 5c, 6g and 10

Compounds 5c, 6g and 10 resulted to be very potent in inhibiting mPTP in the in vitro tests. In order to identify any side effects in the use of these compounds in living cells that could exclude their future development in therapy, and given the crucial role of the C subunit of the ATP synthase in energy production, two important parameters were evaluated, such as cell viability and mitochondrial ATP content, in basal conditions and following Ca²⁺-dependent stimulus (Figure 5A-C). Treatment of the cells with micromolar concentrations of 5c, 6g and 10 for 24h and 48h did not induce a significant reduction of 3-(4,5-dimethylthiazol-2-yl)-2,5-diphenyltetrazolium bromide (MTT) compared to the control. None of the concentrations considered resulted to be toxic in vitro, although some negative effect was perceived after 72h (Figure 5C).

Compound 1, similarly to Oligomycin, significantly inhibited the proton flow and the ATP synthesis, as established by the luciferin-luciferase assay (28683062). Surprisingly, 5c, 6g and 10 gave positive results since, by binding to the C subunit and inhibiting mPTP, they did not reduce ATP levels in vitro.

The opening of mPTP is known to play a key role in the release of cytochrome C (Cyt.C) from the mitochondria to the cytosol (15800627). For this reason, the ability of 5c, 6g and 10 to counteract the release of this protein from mitochondrial crests was also investigated. Figure 5D shows the protective action of selected compounds from the study as, under Ca²⁺-dependent stress conditions, they prevent the release of Cyt.C from the mitochondria, and therefore the activation of the apoptotic cascade.

### Example 11: Cardioprotective effect of compound 10 in rat isolated heart

Considering the potential of the inhibitors claimed herein in relation to: i) ability to desensitize the opening of mPTP; ii) ability to protect against cell death by inhibiting the release of Cyt.C; iii) ability to preserve cell viability, and iv) mitochondrial ATP content, the effects of compound 10 were studied in an animal model of cardiac ischemia-reperfusion. Beating rat hearts were isolated and inserted into a Langendorff system, under continuous perfusion with Krebs Henseleit Buffer (KHB) at 37 °C, in the presence of oxygen. The ex vivo protocol included 20 minutes of heart stabilization, then the retrograde perfusion was progressively stopped to induce 30 minutes of general ischemia followed by 1 hour of reperfusion (Figure 6A). As indicated in panel A, compound 10 was administered in the reperfusion phase during the first 10 minutes of reperfusion. The dose of compound 10 was chosen based on previous experiments aimed at screening and determining the highest and not cytotoxic dose to be infused into the heart. In isolated hearts, constant-volume perfusion of compound 10 at 10 µM resulted in a decrease in coronary perfusion pressure (CPP) and diastolic pressure (EDP) with increased diastolic pressure of the left ventricle (LVEDP), which summed up a reduction in muscle stiffness, vasoconstriction and deterioration of myocardial performance (Figure 6B-D). At the end of the procedure, the hearts were also analyzed for cell death by terminal deoxynucleotidyl transferase dUTP nick-end labelling (TUNEL) assay. In the rat heart subjected to reperfusion injury, 64% of cardiomyocytes resulted to be positive for TUNEL and were therefore classified as apoptotic, a percentage that was significantly reduced in the presence of compound 10 at 10µM) (Figure 6E).

## Claims

1. A 1,3,8-triazaspiro compound of Formula (I): 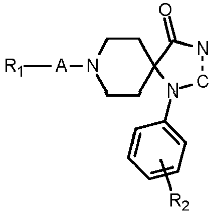 wherein
A is -CH₂, -SO₂-, -NH-CO-, -NH-CS- or -CO-;
the dashed line represents a single or double bond;
R₁ is a substituent selected from the group consisting of (C₁-C₃) alkyl, phenyl, thienyl and cyclohexyl, being said substituent optionally substituted by halogen or (C₁-C₃) alkyl; and
R₂ is a substituent selected from the group consisting of H, (C₁-C₃) alkyl, (C₁-C₃) alkoxy, -CF₃ and halogen; and
wherein
when the dashed line is a double bond, A is -CH₂- and R₁ is phenyl,
or a pharmaceutically acceptable salt thereof
for use in the treatment of reperfusion injury diseases.

2. The 1,3,8-triazaspiro compound for use according to claim 1, wherein the compound is a selective inhibitor of the C subunit of the F₁/F₀-ATP synthase complex and a modulator of the mitochondrial permeability transition pore activity in mammalian cells and tissues, in the treatment of reperfusion injury diseases.

3. The 1,3,8-triazaspiro compound for use according to claim 1 or 2, wherein the reperfusion injury diseases are cardiac, neurological and nephrological diseases.

4. The 1,3,8-triazaspiro compound for use according to claim 1 or 2, wherein said reperfusion injury diseases are cardiac and nephrological diseases deriving from ischemic events related to heart attack and stroke.

5. The 1,3,8-triazaspiro compound for use according to any one of claims 1-4,
wherein A is -SO₂-, the dashed line is a single bond, R₁ is selected from the group consisting of phenyl optionally substituted by halogen or (C₁-C₃) alkyl, thienyl optionally substituted by halogen or (C₁-C₃) alkyl, and R₂ is hydrogen or halogen.

6. The 1,3,8-triazaspiro compound for use according to any one of claims 1-4,
wherein A is -CO-, the dashed line is a single bond, R₁ is selected from the group consisting of phenyl and (C₁-C₃) alkyl, and R₂ is hydrogen.

7. The 1,3,8-triazaspiro compound for use according to any one of claims 1-4,
wherein A is -NH-CO- or -NHCS-, the dashed line is a single bond, R₁ is selected from the group consisting of phenyl and cyclohexyl, and R₂ is hydrogen.

8. The 1,3,8-triazaspiro compound for use according to any one of claims 1-4,
wherein A is -CH₂-, the dashed line is a single or double bond, R₁ is phenyl, and R₂ is hydrogen, halogen, (C₁-C₃) alkyl, (C₁-C₃) alkoxy or -CF₃.

9. The 1,3,8-triazaspiro compound for use according to any one of claims 1-4
selected from the group consisting of:
8-benzyl-1-phenyl-1,3,8-triazaspiro[4.5]dec-2-en-4-one (compound 5a),
8-benzyl-1-(4-fluorophenyl)-1,3,8-triazaspiro[4.5]dec-2-en-4-one (compound 5b),
8-benzyl-1-(4-chlorophenyl)-1,3,8-triazaspiro[4.5]dec-2en-4-one (compound 5c),
8-benzyl-1-(p-tosyl)-1,3,8-triazaspiro[4.5]dec-2-en-4-one (compound 5d),
8-benzyl-1-(4-isopropylphenyl)-1,3,8-triazaspiro[4.5]dec-2-en-4-one (compound 5e),
8-benzyl-1-(4-methoxyphenyl)-1,3,8-triazaspiro[4.5]dec-2-en-4-one (compound 5f),
8-benzyl-1-(3-(trifluoromethyl)phenyl)-1,3,8-triazaspiro[4.5]dec-2-en-4-one (compound 5g),
8-benzyl-1-phenyl-1,3,8-triazaspiro[4.5]decan-4-one (compound 6a),
8-benzyl-1-(4-fluorophenyl)-1,3,8-triazaspiro[4.5]decan-4-one (compound 6b),
8-benzyl-1-(4-chlorophenyl)-1,3,8-triazaspiro[4.5]decan-4-one (compound 6c),
8-benzyl-1-(p-tosyl)-1,3,8-triazaspiro[4.5]decan-4-one (compound 6d),
8-benzyl-1-(4-isopropylphenyl)-1,3,8-triazaspiro[4.5]decan-4-one (compound 6e),
8-benzyl-1-(4-methoxyphenyl)-1,3,8 triazaspiro[4.5]decan-4-one (compound 6f),
8-benzyl-1-(3-(trifluoromethyl)phenyl-1,3,8-triazaspiro[4.5]decan-4-one (compound 6g),
1-phenyl-8-tosyl-1,3,8-triazaspiro[4.5]decan-4-one (compound 1),
1-phenyl-8-mesyl-1,3,8-triazaspiro[4.5]decan-4-one (compound 8),
8-((4-fluorophenyl)sulfonyl)-1-phenyl-1,3,8-triazaspiro[4.5]decan-4-one (compound 9),
8-((5-chlorothiophen-2-yl)sulfonyl)-1-phenyl-1,3,8-triazaspiro[4.5]decan-4-one (compound 10),
1-(4-fluorophenyl)-8-((4-fluorophenyl)sulfonyl)-1,3,8-triazaspiro[4.5]decan-4-one (compound 11),
8-((5-chlorothiophen-2-yl)sulfonyl)-1-(4-fluorophenyl)-1,3,8-triazaspiro [4.5]decan-4-one (compound 12),
8-acetyl-1-phenyl-1,3,8-triazaspiro[4.5]decan-4-one (compound 13),
8-benzoyl-1-phenyl-1,3,8-triazaspiro[4.5]decan-4-one (compound 14),
4-oxo-N-1-diphenyl-1,3,8-triazaspiro[4.5]decane-8-carboxamide (compound 15),
N-cyclohexyl-4-oxo-1-phenyl-1,3,8-triazaspiro[4.5]decane-8-carboxamide (compound 16), and
N-cyclohexyl-4-oxo-1-phenyl-1,3,8-triazaspiro[4.5]decane-8carbothioamide (compound 17).

10. A 1,3,8-triazaspiro compound of Formula (I): 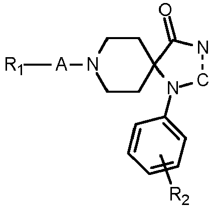 wherein
A is -CH₂, -SO₂-, -NH-CO-, -NH-CS- or -CO-;
the dashed line represents a single or double bond;
R₁ is a substituent selected from the group consisting of (C₁-C₃) alkyl, phenyl, thienyl and cyclohexyl, being said substituent optionally substituted by halogen or (C₁-C₃) alkyl; and
R₂ is a substituent selected from the group consisting of H, (C₁-C₃) alkyl, (C₁-C₃) alkoxy, -CF₃ and halogen; and
wherein
when A is -CH₂, R₁ is phenyl, and R₂ is isopropyl, or
when A is -CH₂, R₁ is phenyl, and R₂ is methoxy, then the dashed line is a double bond
for use as a medicament.

11. The 1,3,8-triazaspiro compound for use according to claim 10 selected from the group consisting of:
8-benzyl-1-(4-isopropylphenyl)-1,3,8-triazaspiro[4.5]dec-2-en-4-one (compound 5e),
8-benzyl-1-(4-methoxyphenyl)-1,3,8-triazaspiro[4.5]dec-2-en-4-one (compound 5f),
8-benzyl-1-(4-isopropylphenyl)-1,3,8-triazaspiro[4.5]decan-4-one (compound 6e),
8-benzyl-1-(4-methoxyphenyl)-1,3,8-triazaspiro[4.5]decan-4-one (compound 6f),
1-phenyl-8-tosyl-1,3,8-triazaspiro[4.5]decan-4-one (compound 1),
1-phenyl-8-mesyl-1,3,8-triazaspiro[4.5]decan-4-one (compound 8),
8-((4-fluorophenyl)sulfonyl)-1-phenyl-1,3,8-triazaspiro[4.5]decan-4-one (compound 9),
8-((5-chlorothiophen-2-yl)sulfonyl)-1-phenyl-1,3,8-triazaspiro[4.5]decan-4-one (compound 10),
1-(4-fluorophenyl)-8-((4-fluorophenyl)sulfonyl)-1,3,8-triazaspiro[4.5]decan-4-one (compound 11),
8-((5-chlorothiophen-2-yl)sulfonyl)-1-(4-fluorophenyl)-1,3,8-triazaspiro[4.5]decan-4-one (compound 12),
8-acetyl-1-phenyl-1,3,8-triazaspiro[4.5]decan-4-one (compound 13),
8-benzoyl-1-phenyl-1,3,8-triazaspiro[4.5]decan-4-one (compound 14),
4-oxo-N-1-diphenyl-1,3,8-triazaspiro[4.5]decane-8-carboxamide (compound 15),
N-cyclohexyl-4-oxo-1-phenyl-1,3,8-triazaspiro[4.5]decane-8-carboxamide (compound 16), and
N-cyclohexyl-4-oxo-1-phenyl-1,3,8-triazaspiro[4.5]decane-8-carbothioamide (compound 17).

12. A 1,3,8-triazaspiro compound of Formula (I): 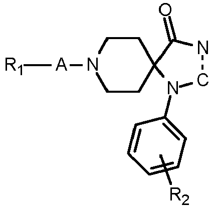 wherein
A is -CH₂-, -SO₂-, -NH-CO-, or -NH-CS-;
the dashed line represents a single or double bond;
R₁ is a substituent selected from the group consisting of (C₁-C₃) alkyl, phenyl, thienyl and cyclohexyl, being said substituent optionally substituted by halogen or (C₁-C₃) alkyl;
and
R₂ is a substituent selected from the group consisting of H, (C₁-C₃) alkyl, (C₁-C₃) alkoxy and halogen; and
wherein
when A is -CH₂- and the dashed line is a single bond, R₁ is phenyl, and R₂ is isopropyl,
when A is -CH₂- and the dashed line is a double bond, R₁ is phenyl, and R₂ is isopropyl or methoxy, and
when A is -SO₂-, R₁ is methyl and R₂ is hydrogen, or R₁ is phenyl or thienyl substituted with halogen and R₂ is hydrogen or halogen.

13. The compound according to claim 12, selected from the group consisting of
8-benzyl-1-(4-isopropylphenyl)-1,3,8-triazaspiro[4.5]dec-2-en-4-one (compound 5e),
8-benzyl-1-(4-methoxyphenyl)-1,3,8-triazaspiro[4.5]dec-2-en-4-one (compound 5f),
8-benzyl-1-(4-isopropylphenyl)-1,3,8-triazaspiro[4.5]decan-4-one (compound 6e),
8-benzyl-1-(4-methoxyphenyl)-1,3,8-triazaspiro[4.5]decan-4-one (compound 6f),
1-phenyl-8-mesyl-1,3,8-triazaspiro[4.5]decan-4-one (compound 8),
1-(4-fluorophenyl)-8-((4-fluorophenyl)sulfonyl)-1,3,8-triazaspiro[4.5]decan-4-one (compound 11),
8-((5-chlorothiophen-2-yl)sulfonyl)-1-(4-fluorophenyl)-1,3,8-triazaspiro[4.5]decan-4-one (compound 12),
4-oxo-N-1-diphenyl-1,3,8-triazaspiro[4.5]decane-8-carboxamide (compound 15),
N-cyclohexyl-4-oxo-1-phenyl-1,3,8-triazaspiro[4.5]decane-8-carboxamide (compound 16), and
N-cyclohexyl-4-oxo-1-phenyl-1,3,8-triazaspiro[4.5]decane-8-carbothioamide (compound 17).

14. The compound for use according to any one of claims 1-4 or claim 10, wherein A is -SO₂- or -CH₂-.

15. The compound for use according to any one of claims 1-4 or claim 10, wherein the pharmaceutically acceptable salt is selected from the group consisting of hydrochloride, hydrobromide, sulphate, phosphate, acetate, succinate, oxalate, ascorbate, tartrate, gluconate, benzoate, maleate, fumarate, and stearate.

16. A pharmaceutical composition comprising a compound for use according to any one of claims 10-11 and pharmacologically acceptable excipients, for the treatment of diseases and disorders or conditions associated with the need to induce cardioprotection, wherein the diseases and disorders or conditions associated with the need to induce cardioprotection include heart attack and ischemia-reperfusion injury.

## Patentansprüche

1. 1,3,8-Triazaspiro-Verbindung der Formel (I): 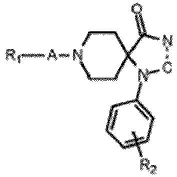 wobei
A -CH₂, -SO₂-, -NH-CO-, -NH-CS- oder -CO- ist;
die gestrichelte Linie eine Einfach- oder Doppelbindung darstellt;
R₁ ein Substituent ist, ausgewählt aus der Gruppe bestehend aus (C₁-C₃) Alkyl, Phenyl, Thienyl und Cyclohexyl, wobei dieser Substituent gegebenenfalls durch Halogen oder (C₁-C₃) Alkyl substituiert ist; und
R₂ ein Substituent ist, ausgewählt aus der Gruppe bestehend aus H, (C₁-C₃) Alkyl, (C₁-C₃) Alkoxy, -CF₃ und Halogen; und
wobei
wenn die gestrichelte Linie eine Doppelbindung ist, A -CH₂- ist und R₁ Phenyl ist,
oder ein pharmazeutisch annehmbares Salz davon
für die Behandlung von Krankheiten mit Reperfusionsschäden.

2. 1,3,8-Triazaspiro-Verbindung zur Verwendung nach Anspruch 1, wobei die Verbindung ein selektiver Inhibitor der C-Untereinheit des F₁/F₀-ATP-Synthase-Komplexes und ein Modulator der mitochondrialen Permeabilitätsübergangsporenaktivität in Säugetierzellen und -geweben bei der Behandlung von Krankheiten mit Reperfusionsschäden ist.

3. 1,3,8-Triazaspiro-Verbindung zur Verwendung nach Anspruch 1 oder 2, wobei die Krankheiten mit Reperfusionsschäden kardiale, neurologische und nephrologische Krankheiten sind.

4. 1,3,8-Triazaspiro-Verbindung zur Verwendung nach Anspruch 1 oder 2, wobei die Krankheiten mit Reperfusionsschäden kardiale und nephrologische Krankheiten sind, die von ischämischen Ereignissen im Zusammenhang mit Herzinfarkt und Schlaganfall herrühren.

5. 1,3,8-Triazaspiro-Verbindung zur Verwendung nach einem der Ansprüche 1 bis 4, wobei A -SO₂- ist, die gestrichelte Linie eine Einfachbindung ist, R₁ ausgewählt ist aus der Gruppe bestehend aus Phenyl, gegebenenfalls substituiert durch Halogen oder (C₁-C₃) Alkyl, Thienyl, gegebenenfalls substituiert durch Halogen oder (C₁-C₃) Alkyl, und R₂ Wasserstoff oder Halogen ist.

6. 1,3,8-Triazaspiro-Verbindung zur Verwendung nach einem der Ansprüche 1 bis 4, wobei A -CO- ist, die gestrichelte Linie eine Einfachbindung ist, R₁ ausgewählt ist aus der Gruppe bestehend aus Phenyl und (C₁-C₃) Alkyl, und R₂ Wasserstoff ist.

7. 1,3,8-Triazaspiro-Verbindung zur Verwendung nach einem der Ansprüche 1 bis 4, wobei A -NH-CO- oder -NHCS- ist, die gestrichelte Linie eine Einfachbindung ist, R₁ ausgewählt ist aus der Gruppe bestehend aus Phenyl und Cyclohexyl R₂ Wasserstoff ist.

8. 1,3,8-Triazaspiro-Verbindung zur Verwendung nach einem der Ansprüche 1 bis 4, worin A -CH₂- ist, die gestrichelte Linie eine Einfach- oder Doppelbindung ist, R₁ Phenyl ist und R₂ Wasserstoff, Halogen, (C₁-C₃) Alkyl, (C₁-C₃) Alkoxy oder -CF₃ ist.

9. 1,3,8-Triazaspiro-Verbindung zur Verwendung nach einem der Ansprüche 1 bis 4, ausgewählt aus der Gruppe bestehend aus:
8-Benzyl-1-phenyl-1,3,8-triazaspiro[4.5]dec-2-en-4-one (Verbindung 5a),
8-Benzyl-1-(4-fluorophenyl)-1,3,8-triazaspiro[4.5]dec-2-en-4-one (Verbindung 5b),
8-Benzyl-1-(4-chlorophenyl)-1,3,8-triazaspiro[4.5]dec-2en-4-one (Verbindung 5c),
8-Benzyl-1-(p-tosyl)-1,3,8-triazaspiro[4.5]dec-2-en-4-one (Verbindung 5d),
8-Benzyl-1-(4-isopropylphenyl)-1,3,8-triazaspiro[4.5]dec-2-en-4-one (Verbindung 5e),
8-Benzyl-1-(4-methoxyphenyl)-1,3,8-triazaspiro[4.5]dec-2-en-4-one (Verbindung 5f),
8-Benzyl-1-(3-(trifluormethyl)phenyl)-1,3,8-triazaspiro[4.5]dec-2-en-4-on (Verbindung 5g),
8-Benzyl-1-phenyl-1,3,8-triazaspiro[4.5]decan-4-on (Verbindung 6a),
8-Benzyl-1-(4-fluorphenyl)-1,3,8-triazaspiro[4.5]decan-4-on (Verbindung 6b),
8-Benzyl-1-(4-chlorphenyl)-1,3,8-triazaspiro[4.5]decan-4-on (Verbindung 6c),
8-Benzyl-1-(p-tosyl)-1,3,8-triazaspiro[4.5]decan-4-on (Verbindung 6d),
8-Benzyl-1-(4-isopropylphenyl)-1,3,8-triazaspiro[4.5]decan-4-on (Verbindung 6e),
8-Benzyl-1-(4-methoxyphenyl)-1,3,8-triazaspiro[4.5]decan-4-on (Verbindung 6f),
8-Benzyl-1-(3-(trifluormethyl)phenyl-1,3,8-triazaspiro[4.5]decan-4-on (Verbindung 6g),
1-Phenyl-8-tosyl-1,3,8-triazaspiro[4.5]decan-4-one (Verbindung 1),
1-Phenyl-8-mesyl-1,3,8-triazaspiro[4.5]decan-4-one (Verbindung 8),
8-((4-Fluorphenyl)sulfonyl)-1-phenyl-1,3,8-triazaspiro[4.5]decan-4-on (Verbindung 9),
8-((5-Chlorthiophen-2-yl)sulfonyl)-1-phenyl-1,3,8-triazaspiro[4.5]decan-4-on (Verbindung 10),
1-(4-Fluorphenyl)-8-((4-Fluorphenyl)sulfonyl)-1,3,8-triazaspiro[4.5]decan-4-on (Verbindung 11),
8-((5-Chlorthiophen-2-yl)sulfonyl)-1-(4-fluorphenyl)-1,3,8-triazaspiro [4.5]decan-4-on (Verbindung 12),
8-Acetyl-1-phenyl-1,3,8-triazaspiro[4.5]decan-4-one (Verbindung 13),
8-Benzoyl-1-phenyl-1,3,8-triazaspiro[4.5]decan-4-one (Verbindung 14),
4-Oxo-N-1-diphenyl-1,3,8-triazaspiro[4.5]decan-8-carboxamid (Verbindung 15),
N-Cyclohexyl-4-oxo-1-phenyl-1,3,8-triazaspiro[4.5]decan-8-carboxamid (Verbindung 16), und
N-Cyclohexyl-4-oxo-1-phenyl-1,3,8-triazaspiro[4.5]decan-8carbothioamid (Verbindung 17).

10. 1,3,8-Triazaspiro-Verbindung der Formel (I): 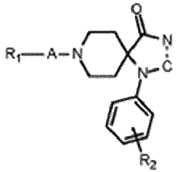 wobei
A -CH₂, -SO₂-, -NH-CO-, -NH-CS- oder -CO- ist;
die gestrichelte Linie eine Einfach- oder Doppelbindung darstellt;
R₁ ein Substituent ist, ausgewählt aus der Gruppe bestehend aus (C₁-C₃) Alkyl, Phenyl, Thienyl und Cyclohexyl, wobei dieser Substituent gegebenenfalls durch Halogen oder (C₁-C₃) Alkyl substituiert ist; und
R₂ ein Substituent ist, ausgewählt aus der Gruppe bestehend aus H, (C₁-C₃) Alkyl, (C₁-C₃) Alkoxy, -CF₃ und Halogen; und
wobei
wenn A -CH₂ ist, R₁ Phenyl ist und R₂ Isopropyl ist, oder
wenn A -CH₂ ist, R₁ Phenyl ist und R₂ Methoxy ist, dann ist die gestrichelte Linie eine Doppelbindung
zur Verwendung als Arzneimittel.

11. 1,3,8-Triazaspiro-Verbindung zur Verwendung nach Anspruch 10, ausgewählt aus der Gruppe bestehend aus:
8-Benzyl-1-(4-isopropylphenyl)-1,3,8-triazaspiro[4.5]dec-2-en-4-one (Verbindung 5e),
8-Benzyl-1-(4-methoxyphenyl)-1,3,8-triazaspiro[4.5]dec-2-en-4-one (Verbindung 5f),
8-Benzyl-1-(4-isopropylphenyl)-1,3,8-triazaspiro[4.5]decan-4-on (Verbindung 6e),
8-Benzyl-1-(4-methoxyphenyl)-1,3,8-triazaspiro[4.5]decan-4-on (Verbindung 6f),
1-Phenyl-8-tosyl-1,3,8-triazaspiro[4.5]decan-4-one (Verbindung 1),
1-Phenyl-8-mesyl-1,3,8-triazaspiro[4.5]decan-4-one (Verbindung 8),
8-((4-Fluorphenyl)sulfonyl)-1-phenyl-1,3,8-triazaspiro[4.5]decan-4-on (Verbindung 9),
8-((5-Chlorthiophen-2-yl)sulfonyl)-1-phenyl-1,3,8-triazaspiro[4.5]decan-4-on (Verbindung 10),
1-(4-Fluorphenyl)-8-((4-Fluorphenyl)sulfonyl)-1,3,8-triazaspiro[4.5]decan-4-on (Verbindung 11),
8-((5-Chlorthiophen-2-yl)sulfonyl)-1-(4-fluorphenyl)-1,3,8-triazaspiro[4.5]decan-4-on (Verbindung 12),
8-Acetyl-1-phenyl-1,3,8-triazaspiro[4.5]decan-4-one (Verbindung 13),
8-Benzoyl-1-phenyl-1,3,8-triazaspiro[4.5]decan-4-one (Verbindung 14),
4-Oxo-N-1-diphenyl-1,3,8-triazaspiro[4.5]decan-8-carboxamid (Verbindung 15),
N-Cyclohexyl-4-oxo-1-phenyl-1,3,8-triazaspiro[4.5]decan-8-carboxamid (Verbindung 16) und
N-Cyclohexyl-4-oxo-1-phenyl-1,3,8-triazaspiro[4.5]decan-8-carbothioamid (Verbindung 17).

12. 1,3,8-Triazaspiro-Verbindung der Formel (I): 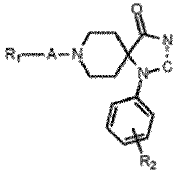 wobei
A -CH₂-, -SO₂-, -NH-CO-, oder -NH-CS- ist;
die gestrichelte Linie eine Einfach- oder Doppelbindung darstellt;
R₁ ein Substituent ist, ausgewählt aus der Gruppe bestehend aus (C₁-C₃) Alkyl, Phenyl, Thienyl und Cyclohexyl, wobei dieser Substituent gegebenenfalls durch Halogen oder (C₁-C₃) Alkyl substituiert ist;
und
R₂ ein Substituent ist, ausgewählt aus der Gruppe bestehend aus H, (C₁-C₃) Alkyl, (C₁-C₃) Alkoxy und Halogen; und
wobei
wenn A -CH₂- ist und die gestrichelte Linie eine Einfachbindung ist, R₁ Phenyl ist und R₂ Isopropyl ist,
wenn A -CH₂- ist und die gestrichelte Linie eine Doppelbindung ist, R₁ Phenyl ist und R₂ Isopropyl oder Methoxy ist, und
wenn A -SO₂- ist, R₁ Methyl ist und R₂ Wasserstoff ist oder R₁ Phenyl oder Thienyl ist, substituiert mit Halogen, und R₂ Wasserstoff oder Halogen ist.

13. Verbindung nach Anspruch 12, ausgewählt aus der Gruppe bestehend aus
8-Benzyl-1-(4-isopropylphenyl)-1,3,8-triazaspiro[4.5]dec-2-en-4-one (Verbindung 5e),
8-Benzyl-1-(4-methoxyphenyl)-1,3,8-triazaspiro[4.5]dec-2-en-4-one (Verbindung 5f),
8-Benzyl-1-(4-isopropylphenyl)-1,3,8-triazaspiro[4.5]decan-4-on (Verbindung 6e),
8-Benzyl-1-(4-methoxyphenyl)-1,3,8-triazaspiro[4.5]decan-4-on (Verbindung 6f),
1-Phenyl-8-mesyl-1,3,8-triazaspiro[4.5]decan-4-one (Verbindung 8),
1-(4-Fluorphenyl)-8-((4-Fluorphenyl)sulfonyl)-1,3,8-triazaspiro[4.5]decan-4-on (Verbindung 11),
8-((5-Chlorthiophen-2-yl)sulfonyl)-1-(4-fluorphenyl)-1,3,8-triazaspiro[4.5]decan-4-on (Verbindung 12),
4-Oxo-N-1-diphenyl-1,3,8-triazaspiro[4.5]decan-8-carboxamid (Verbindung 15),
N-Cyclohexyl-4-oxo-1-phenyl-1,3,8-triazaspiro[4.5]decan-8-carboxamid (Verbindung 16), und
N-Cyclohexyl-4-oxo-1-phenyl-1,3,8-triazaspiro[4.5]decan-8-carbothioamid (Verbindung 17).

14. Verbindung zur Verwendung nach einem der Ansprüche 1 bis 4 oder Anspruch 10, wobei A -SO2- oder -CH₂- ist.

15. Verbindung zur Verwendung nach einem der Ansprüche 1 bis 4 oder Anspruch 10, wobei das pharmazeutisch verträgliche Salz ausgewählt ist aus der Gruppe bestehend aus Hydrochlorid, Hydrobromid, Sulfat, Phosphat, Acetat, Succinat, Oxalat, Ascorbat, Tartrat, Gluconat, Benzoat, Maleat, Fumarat und Stearat.

16. Pharmazeutische Zusammensetzung, umfassend eine Verbindung zur Verwendung nach einem der Ansprüche 10 bis 11 und pharmakologisch annehmbare Hilfsstoffe, zur Behandlung von Krankheiten und Störungen oder Zuständen, die mit der Notwendigkeit verbunden sind, eine Kardioprotektion zu induzieren, wobei die Krankheiten und Störungen oder Zustände, die mit der Notwendigkeit verbunden sind, eine Kardioprotektion zu induzieren, einen Herzinfarkt und einen Ischämie-Reperfusionsschaden umfassen.

## Revendications

1. Composé de 1,3,8-triazaspiro de Formule (I) : 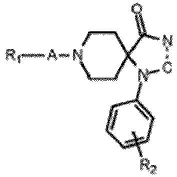 dans lequel
A est -CH₂, -SO₂-, -NH-CO-, -NH-CS- ou -CO- ;
la ligne pointillée représente une liaison simple ou double ;
R₁ est un substituant choisi dans le groupe constitué d'alkyle en(C₁-C₃), de phényle, de thiényle et de cyclohexyle, ledit substituant étant éventuellement substitué par un halogène ou un alkyle en (C₁-C₃) ; et
R₂ est un substituant choisi dans le groupe constitué de H, d'alkyle en (C₁-C₃), d'alcoxy en (C₁-C₃), -CF₃ et d'halogène ; et
dans lequel
lorsque la ligne pointillée est une double liaison, A est -CH₂- et R₁ est un phényle,
ou un sel pharmaceutique acceptable de ceux-ci
pour le traitement des maladies liées aux lésions de reperfusion.

2. Composé 1,3,8-triazaspiro utilisé selon la revendication 1, dans lequel le composé est un inhibiteur sélectif de la sous-unité C du complexe F₁/F₀-ATP synthase et un modulateur de l'activité de pore de transition de perméabilité mitochondriale dans les cellules et les tissus de mammifères, dans le traitement des maladies liées aux lésions de reperfusion.

3. Composé 1,3,8-triazaspiro utilisé selon la revendication 1 ou 2, dans lequel les maladies liées aux lésions de reperfusion sont des maladies cardiaques, neurologiques et néphrologiques.

4. Composé 1,3,8-triazaspiro utilisé selon la revendication 1 ou 2, dans lequel lesdites maladies liées aux lésions de reperfusion sont des maladies cardiaques et néphrologiques résultant d'événements ischémiques liés à des crises cardiaques et à des accidents vasculaires cérébraux.

5. Composé 1,3,8-triazaspiro utilisé selon l'une quelconque des revendications 1 à 4, dans lequel A est -SO₂-, la ligne pointillée est une liaison simple, R₁ est choisi dans le groupe constitué de : phényle éventuellement substitué par l'halogène ou l'alkyle en (C₁-C₃), le thiényle éventuellement substitué par l'halogène ou l'alkyle en (C₁-C₃), et R₂ est de l'hydrogène ou de l'halogène.

6. Composé 1,3,8-triazaspiro utilisé selon l'une quelconque des revendications 1 à 4, dans lequel A est -CO-, la ligne pointillée est une liaison simple, R₁ est choisi dans le groupe constitué de phényle et d'alkyle en (C₁-C₃), et R₂ est de l'hydrogène.

7. Composé 1,3,8-triazaspiro utilisé selon l'une quelconque des revendications 1 à 4, dans lequel A est -NH-CO- ou -NHCS-, la ligne pointillée est une liaison simple, R₁ est choisi dans le groupe constitué de phényle et de cyclohexyle, et R₂ est de l'hydrogène.

8. Composé 1,3,8-triazaspiro utilisé selon l'une quelconque des revendications 1 à 4, dans lequel A est -CH₂-, la ligne pointillée est une liaison simple ou double, R₁ est un phényle, et R₂ est un hydrogène, un halogène, un alkyle en (C₁-C₃), un alcoxy en (C₁-C₃) ou -CF₃.

9. Composé 1,3,8-triazaspiro utilisé selon l'une quelconque des revendications 1 à 4, choisi dans le groupe constitué de :
8-benzyl-1-phényl-1,3,8-triazaspiro[4.5]dec-2-en-4-one (composé 5a),
8-benzyl-1-(4-fluorophényl)-1,3,8-triazaspiro[4.5]dec-2-en-4-one (composé 5b),
8-benzyl-1-(4-chlorophényl)-1,3,8-triazaspiro[4.5]dec-2en-4-one (composé 5c),
8-benzyl-1-(p-tosyl)-1,3,8-triazaspiro[4.5]dec-2-en-4-one (composé 5d),
8-benzyl-1-(4-isopropylphényl)-1,3,8-triazaspiro[4.5]dec-2-en-4-one (composé 5e),
8-benzyl-1-(4-méthoxyphényl)-1,3,8-triazaspiro[4.5]dec-2-en-4-one (composé 5f),
8-benzyl-1-(3-(trifluorométhyl)phényl)-1,3,8-triazaspiro[4.5]dec-2-en-4-one (composé 5g),
8-benzyl-1-phényl-1,3,8-triazaspiro[4.5]décan-4-one (composé 6a),
8-benzyl-1-(4-fluorophényl)-1,3,8-triazaspiro[4.5]décan-4-one (composé 6b),
8-benzyl-1-(4-chlorophényl)-1,3,8-triazaspiro[4.5]décan-4-one (composé 6c),
8-benzyl-1-(p-tosyl)-1,3,8-triazaspiro[4.5]décan-4-one (composé 6d),
8-benzyl-1-(4-isopropylphényl)-1,3,8-triazaspiro[4.5]décan-4-one (composé 6e),
8-benzyl-1-(4-méthoxyphényl)-1,3,8-triazaspiro[4.5]décan-4-one (composé 6f),
8-benzyl-1-(3-(trifluorométhyl)phényl-1,3,8-triazaspiro[4.5]décan-4-one (composé 6g),
1-phényl-8-tosyl-1,3,8-triazaspiro[4.5]décan-4-one (composé 1),
1-phényl-8-mésyl-1,3,8-triazaspiro[4.5]décan-4-one (composé 8),
8-((4-fluorophényl)sulfonyl)-1-phényl-1,3,8-triazaspiro[4.5]décan-4-one (composé 9),
8-((5-chlorothiophène-2-yl)sulfonyl)-1-phényl-1,3,8-triazaspiro[4.5]décan-4-one (composé 10),
1-(4-fluorophényl)-8-((4-fluorophényl)sulfonyl)-1,3,8-triazaspiro[4.5]décan-4-one (composé 11),
8-((5-chlorothiophène-2-yl)sulfonyl)-1-(4-fluorophényl)-1,3,8-triazaspiro [4.5]décan-4-one (composé 12),
8-acétyl-1-phényl-1,3,8-triazaspiro[4.5]décan-4-one (composé 13),
8-benzoyl-1-phényl-1,3,8-triazaspiro[4.5]decan-4-one (composé 14),
4-oxo-N-1-diphényl-1,3,8-triazaspiro[4.5]décane-8-carboxamide (composé 15),
N-cyclohexyl-4-oxo-1-phényl-1,3,8-triazaspiro[4.5]décane-8-carboxamide (composé 16), et
N-cyclohexyl-4-oxo-1-phényl-1,3,8-triazaspiro[4.5]décane-8carbothioamide (composé 17).

10. Composé de 1,3,8-triazaspiro de Formule (I) : 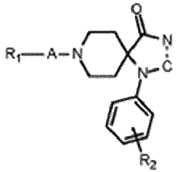 dans lequel
A est -CH₂, -SO₂-, -NH-CO-, -NH-CS- ou -CO- ;
la ligne pointillée représente une liaison simple ou double ;
R₁ est un substituant choisi dans le groupe constitué d'alkyle en(C₁-C₃), de phényle, de thiényle et de cyclohexyle, ledit substituant étant éventuellement substitué par un halogène ou un alkyle en (C₁-C₃) ; et
R₂ est un substituant choisi dans le groupe constitué de H, d'alkyle en (C₁-C₃), d'alcoxy en (C₁-C₃), -CF₃ et d'halogène ; et
dans lequel
lorsque A est -CH₂, R₁ est un phényle et R₂ est un isopropyle, ou
lorsque A est -CH₂, R₁ est un phényle et R₂ est un méthoxy, la ligne pointillée est une double liaison
pour une utilisation en tant que médicament.

11. Composé 1,3,8-triazaspiro utilisé selon la revendication 10, choisi dans le groupe constitué de :
8-benzyl-1-(4-isopropylphényl)-1,3,8-triazaspiro[4.5]dec-2-en-4-one (composé 5e),
8-benzyl-1-(4-méthoxyphényl)-1,3,8-triazaspiro[4.5]dec-2-en-4-one (composé 5f),
8-benzyl-1-(4-isopropylphényl)-1,3,8-triazaspiro[4.5]décan-4-one (composé 6e),
8-benzyl-1-(4-méthoxyphényl)-1,3,8-triazaspiro[4.5]décan-4-one (composé 6f),
1-phényl-8-tosyl-1,3,8-triazaspiro[4.5]décan-4-one (composé 1),
1-phényl-8-mésyl-1,3,8-triazaspiro[4.5]décan-4-one (composé 8),
8-((4-fluorophényl)sulfonyl)-1-phényl-1,3,8-triazaspiro[4.5]décan-4-one (composé 9),
8-((5-chlorothiophène-2-yl)sulfonyl)-1-phényl-1,3,8-triazaspiro[4.5]décan-4-one (composé 10),
1-(4-fluorophényl)-8-((4-fluorophényl)sulfonyl)-1,3,8-triazaspiro[4.5]décan-4-one (composé 11),
8-((5-chlorothiophène-2-yl)sulfonyl)-1-(4-fluorophényl)-1,3,8-triazaspiro[4.5]décan-4-one (composé 12),
8-acétyl-1-phényl-1,3,8-triazaspiro[4.5]décan-4-one (composé 13),
8-benzoyl-1-phényl-1,3,8-triazaspiro[4.5]décan-4-one (composé 14),
4-oxo-N-1-diphényl-1,3,8-triazaspiro[4.5]décane-8-carboxamide (composé 15),
N-cyclohexyl-4-oxo-1-phényl-1,3,8-triazaspiro[4.5]décane-8-carboxamide (composé 16), et
N-cyclohexyl-4-oxo-1-phényl-1,3,8-triazaspiro[4.5]décane-8-carbothioamide (composé 17).

12. Composé de 1,3,8-triazaspiro de Formule (I) : 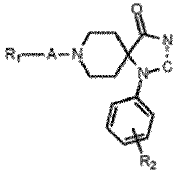 dans lequel
A représente -CH₂-, -SO₂-, -NH-CO-, ou -NH-CS- ;
la ligne pointillée représente une liaison simple ou double ;
R₁ est un substituant choisi dans le groupe constitué d'alkyle en (C₁-C₃), de phényle, de thiényle et de cyclohexyle, ledit substituant étant éventuellement substitué par un halogène ou un alkyle en (C₁-C₃) ;
et
R₂ est un substituant choisi dans le groupe constitué de H, d'alkyle en (C₁-C₃), d'alcoxy en (C₁-C₃) et d'halogène ; et
dans lequel
lorsque A est -CH₂- et que la ligne pointillée est une liaison simple, R₁ est un phényle et R₂ est un isopropyle,
lorsque A est -CH₂- et que la ligne pointillée est une double liaison, R₁ est un phényle et R₂ est un isopropyle ou un méthoxy, et
lorsque A est -SO₂-, R₁ est un méthyle et R₂ est un hydrogène, ou R₁ est un phényle ou un thiényle substitué par un halogène et R₂ est un hydrogène ou un halogène.

13. Composé selon la revendication 12, choisi dans le groupe constitué de
8-benzyl-1-(4-isopropylphényl)-1,3,8-triazaspiro[4.5]dec-2-en-4-one (composé 5e),
8-benzyl-1-(4-méthoxyphényl)-1,3,8-triazaspiro[4.5]dec-2-en-4-one (composé 5f),
8-benzyl-1-(4-isopropylphényl)-1,3,8-triazaspiro[4.5]décan-4-one (composé 6e),
8-benzyl-1-(4-méthoxyphényl)-1,3,8-triazaspiro[4.5]décan-4-one (composé 6f),
1-phényl-8-mésyl-1,3,8-triazaspiro[4.5]décan-4-one (composé 8),
1-(4-fluorophényl)-8-((4-fluorophényl)sulfonyl)-1,3,8-triazaspiro[4.5]décan-4-one (composé 11),
8-((5-chlorothiophène-2-yl)sulfonyl)-1-(4-fluorophényl)-1,3,8-triazaspiro[4.5]décan-4-one (composé 12),
4-oxo-N-1-diphényl-1,3,8-triazaspiro[4.5]décane-8-carboxamide (composé 15),
N-cyclohexyl-4-oxo-1-phényl-1,3,8-triazaspiro[4.5]décane-8-carboxamide (composé 16), et
N-cyclohexyl-4-oxo-1-phényl-1,3,8-triazaspiro[4.5]décane-8-carbothioamide (composé 17).

14. Composé utilisé selon l'une quelconque des revendications 1 à 4 ou la revendication 10, dans lequel A est -SO2- ou -CH₂-.

15. Composé utilisé selon l'une quelconque des revendications 1 à 4 ou la revendication 10, dans lequel le sel pharmaceutiquement acceptable est choisi dans le groupe constitué de : chlorhydrate, bromhydrate, sulfate, phosphate, acétate, succinate, oxalate, ascorbate, tartrate, gluconate, benzoate, maléate, fumarate et stéarate.

16. Composition pharmaceutique comprenant un composé utilisé selon l'une quelconque des revendications 10 à 11 et des excipients pharmacologiquement acceptables, pour le traitement de maladies et de troubles ou d'états associés à la nécessité d'induire une cardioprotection, les maladies et les troubles ou les états associés à la nécessité d'induire une cardioprotection comprenant la crise cardiaque et les lésions d'ischémie-reperfusion.
